(19) Europäisches Patentamt
European Patent Office
Office européen des brevets

(11) Publication number: **0 183 177**
**A1**

(12) # EUROPEAN PATENT APPLICATION

(21) Application number: **85114733.0**

(22) Date of filing: **19.11.85**

(51) Int. Cl.[4]: **C 07 C 103/56**
C 07 C 103/30, A 61 K 31/16
C 07 D 295/18, A 61 K 31/23
A 61 K 31/19, C 07 C 69/732
C 07 C 69/734, C 07 C 59/42
C 07 C 59/46

(30) Priority: **19.11.84 JP 244062/84**

(43) Date of publication of application:
**04.06.86 Bulletin 86/23**

(84) Designated Contracting States:
**AT BE CH DE FR GB IT LI NL SE**

(71) Applicant: **SUMITOMO PHARMACEUTICALS COMPANY, LIMITED**
**40, Dosho-machi 2-chome**
**Higashi-ku Osaka-shi Osaka-fu(JP)**

(72) Inventor: **Nakamura, Toshio**
**8-3-104, Hamakoshien-3-chome**
**Nishinomiya-shi(JP)**

(72) Inventor: **Muraoka, Masami**
**Sumitomo Kagaku Funabashi House 406, 8-1**
**Natsumidai-1-chome Funabashi-shi(JP)**

(72) Inventor: **Ono, Keiichi**
**10-4, Momoyamadai-3-cho**
**Sakai-shi(JP)**

(72) Inventor: **Yamamoto, Michihiro**
**16-40-309, Takagihigashimachi**
**Nishinomiya-shi(JP)**

(74) Representative: **Vossius Vossius Tauchner Heunemann Rauh**
**Siebertstrasse 4 P.O. Box 86 07 67**
**D-8000 München 86(DE)**

(54) **Unsaturated fatty acid derivatives and their production.**

(57) A compound of the formula:

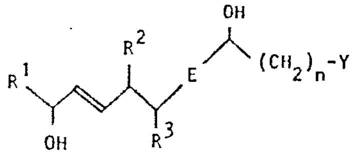

wherein Y is a free or esterified carboxyl group, or a group of the formula:

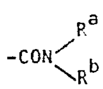

(wherein $R^a$ and $R^b$ are each independently a hydrogen atom, a $C_1$-$C_4$ alkyl group, a $C_3$-$C_7$ cycloalkyl group, a benzyl group, a phenyl group, a phenyl group substituted with a halogen atom or a $C_1$-$C_4$ alkyl group, or, when taken together with the adjacent nitrogen atom, they represent a 5 to 7 membered saturated heterocyclic goup); $R^1$ is a $C_1$-$C_{12}$ alkyl group, a $C_2$-$C_{12}$ alkenyl group, a $C_2$-$C_{12}$ alkynyl group, a $C_3$-$C_{10}$ cycloalkyl group, a $C_4$-$C_{10}$ cycloalkenyl group, a hydroxy $C_1$-$C_{13}$ alkyl group, an $C_1$-$C_{12}$ alkyl group substituted with a group of the formula:

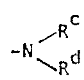

(wherein $R^c$ and $R^d$ are each independently a hydrogen atom, or a $C_1$-$C_4$ alkyl group), a $C_3$-$C_{10}$ heterocyclic group, a phenyl group optionally substituted with one to three substituents selected from the group consisting of halogen atom, hydroxyl group, $C_1$-$C_4$ alkyl group, trifluoromethyl group, $C_1$-$C_4$ alkoxy group, and group of the formula:

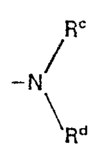

(wherein $R^c$ and $R^d$ are as defined above) or a group of the formula:

./...

EP 0 183 177 A1

A−B

[wherein A is a $C_1$-$C_7$ alkylene chain and B is a $C_3$-$C_{10}$ cycloalkyl group, a $C_4$-$C_{10}$ cycloalkenyl group, a $C_1$-$C_{12}$ alkoxy group, a $C_1$-$C_{12}$ alkylthio group, a $C_3$-$C_{10}$ cycloalkoxy group, a $C_4$-$C_{10}$ cycloalkenyloxy group, a $C_3$-$C_{10}$ heterocyclic group, or a phenyl or phenoxy group optionally substituted with one to three substituents selected from the group consisting of halogen atom, hydroxy group, $C_1$-$C_4$ alkyl group, group of the formula:

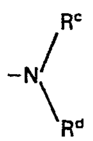

(wherein $R^c$ and $R^d$ are as defined above), trifluoromethyl group, $C_1$-$C_4$ alkylthio group and $C_1$-$C_4$ alkoxy group]; n is 2, 3 or 4; $R^2$ and $R^3$ are hydrogen atoms or, when taken together, they mean a single linkage to form a *trans* double bond between the adjacent carbon atoms, and E is a *cis* or *trans* vinylene group; provided that when n is 3 and $R^1$ is a n-octyl group, a 2-octenyl group or a 2,5-octadienyl group, $R^2$ and $R^3$ are hydrogen atoms, and that when n is 2, E is a *trans* vinylene group or a non-toxic pharmaceutically acceptable salt thereof. Said compound have potent anti-leucotriene $B_4$ action, and are useful in the treatment of inflammation and allergy.

VOSSIUS · VOSSIUS · TAUCHNER
HEUNEMANN · RAUH
PATENTANWÄLTE
SIEBERTSTR. 4, 8000 MÜNCHEN 80
TEL. (089) 47 40 75

Our Ref.: U 148 EP
Case: B282-02
Sumitomo Pharmaceuticals Co., Ltd.
November 19, 1985
0183177

UNSATURATED FATTY ACID DERIVATIVES AND THEIR PRODUCTION

The present invention relates to novel unsaturated fatty acids and their production.

More particularly, this invention relates to novel unsaturated fatty acids, to processes for production thereof and to pharmaceutical compositions containing at least one of the unsaturated fatty acids.

The novel unsaturated fatty acids provided by the present invention are those represented by the formula [I]:

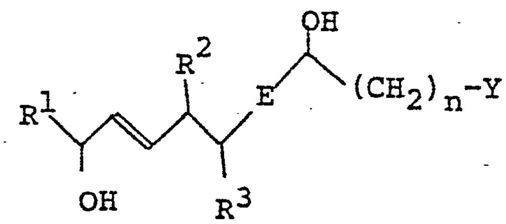

[I]

wherein Y is a free or esterified carboxyl group, or a group of the formula:

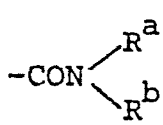

(wherein $R^a$ and $R^b$ are each independently a hydrogen atom, a $C_1$-$C_4$ alkyl group, a $C_3$-$C_7$ cycloalkyl group, a benzyl group, a phenyl group, a phenyl group substituted with a halogen atom or a $C_1$-$C_4$ alkyl group, or, when taken together with the adjacent nitrogen atom, they represent a 5 to 7 membered saturated heterocyclic group); $R^1$ is a $C_1$-$C_{12}$ alkyl group, a $C_2$-$C_{12}$ alkenyl group, a $C_2$-$C_{12}$

alkynyl group, a $C_3$-$C_{10}$ cycloalkyl group, a $C_4$-$C_{10}$ cycloalkenyl group, a hydroxy $C_1$-$C_{12}$ alkyl group, a $C_1$-$C_{12}$ alkyl group substituted with a group of the formula:

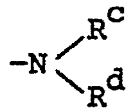

(wherein $R^c$ and $R^d$ are each independently a hydrogen atom, or a $C_1$-$C_4$ alkyl group), a $C_3$-$C_{10}$ heterocyclic group, a phenyl group optionally substituted with one to three substituents selected from the group consisting of halogen atom, hydroxyl group, $C_1$-$C_4$ alkyl group, trifluoromethyl group, $C_1$-$C_4$ alkoxy group, and group of the formula: 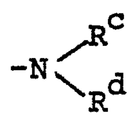 (wherein $R^c$ and $R^d$ are as defined above) or a group of the formula:

A-B

[wherein A is a $C_1$-$C_7$ alkylene chain and B is a $C_3$-$C_{10}$ cycloalkyl group, a $C_4$-$C_{10}$ cycloalkenyl group, a $C_1$-$C_{12}$ alkoxy group, a $C_1$-$C_{12}$ alkylthio group, a $C_3$-$C_{10}$ cycloalkoxy group, a $C_4$-$C_{10}$ cycloalkenyloxy group, a $C_3$-$C_{10}$ heterocyclic group, or a phenyl or phenoxy group optionally substituted with one to three substituents selected from the group consisting of halogen atom, hydroxy group, $C_1$-$C_4$ alkyl group, group of the formula: 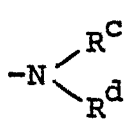 (wherein $R^c$ and $R^d$ are as defined above), tri-

fluoromethyl group, $C_1$-$C_4$ alkylthio group and $C_1$-$C_4$ alkoxy group]; n is 2, 3 or 4; $R^2$ and $R^3$ are hydrogen atoms or, when taken together, they mean a single linkage to form a *trans* double bond between the adjacent carbon atoms, and E is a *cis* or *trans* vinylene group; provided that when n is 3 and $R^1$ is a n-octyl group, a 2-octenyl group or a 2,5-octadienyl group, $R^2$ and $R^3$ are hydrogen atoms, and that when n is 2, E is a *trans* vinylene group.

In the significances as used above, the term "halogen" includes fluorine, chlorine, bromine and iodine; the terms "$C_1$-$C_4$ alkyl", "$C_1$-$C_4$ alkoxy" and "$C_1$-$C_4$ alkylthio" each means a straight or branched chain alkyl, alkoxy or alkylthio group having from 1 to 4 carbon atoms (e.g. methyl, ethyl, n-propyl, isopropyl, n-butyl, methoxy, ethoxy, n-propoxy, methylthio, ethylthio, isopropoxy, n-butoxy, etc.).

The term "$C_1$-$C_{12}$ alkyl" in the following cases, "$C_1$-$C_{12}$ alkyl" and "$C_1$-$C_{12}$ alkyl in the $C_1$-$C_{12}$ alkoxy group" and "$C_1$-$C_{12}$ alkyl in the $C_1$-$C_{12}$ alkylthio group", means a straight or branched chain alkyl group having from one to 12 carbon atoms (e.g. methyl, ethyl, n-propyl, isopropyl, n-butyl, isobutyl, n-pentyl, isopentyl, 1-methylpentyl, 2-methylpentyl, 1,1-dimethylpentyl, 1-ethylpentyl, 2-ethylpentyl, n-hexyl, 1-methylhexyl, 2-methylhexyl, 3-methylhexyl, heptyl, 1-methylheptyl, 2-methylheptyl, 1-ethylheptyl, 2-ethylheptyl, n-octyl, 1-methyloctyl, 2-methyloctyl, 1-ethyloctyl, 2-ethyloctyl, 2,6-dimethylheptyl, 1,6-dimethylheptyl, n-nonyl, 1-methylnonyl, 2-methylnonyl, n-decyl, 1-methyldecyl, 2-methyldecyl, 2-ethyldecyl etc.).

The terms "$C_2$-$C_{12}$ alkenyl" and "$C_2$-$C_{12}$ alkynyl" each means a straight or branched chain alkenyl or alkynyl group having from 2 to 12 carbon atoms (e.g. vinyl, 2-propenyl, 2-butenyl, 2-pentenyl, 2-hexenyl, 5-heptenyl, 6-methyl-5-heptenyl, 2,6-dimethyl-5-heptenyl, 3-pentenyl, 4-pentenyl, 2,6-dimethyl-5-octenyl, 1,1,6-trimethyl-5-heptenyl, 4,8-dimethyl-7-nonenyl, 2,6-dimethyl-1,5-heptadienyl, 2-propynyl, 1-methylenepentyl, 2-butynyl, 2-pentynyl, 3-pentynyl, 1-methyl-3-pentynyl, 4-pentynyl, 4-hexynyl, 5-heptynyl, 6-heptynyl, 2-methyl-5-heptynyl, etc.).

The term "$C_3$-$C_{10}$ cycloalkyl" in the both cases, "$C_3$-$C_{10}$ cycloalkyl" and "$C_3$-$C_{10}$ cycloalkyl in the $C_3$-$C_{10}$ cycloalkoxy group" means a cyclic alkyl group which is optionally substituted with a $C_1$-$C_4$ alkyl or alkenyl group and which has from 3 to 10 carbon atoms (e.g. cyclopropyl, cyclobutyl, cyclopentyl, cyclohexyl, cycloheptyl, 2-isopropylidenemethyl-3,3-dimethylcyclopropyl, 2-propylcyclopropyl, 3-ethylcyclobutyl, 3-ethylcyclopentyl, 4-methylcyclohexyl, 3-ethylcyclohexy, 4-methylcycloheptyl, 2-isopropyl-5-methylcyclohexyl, norbornyl, adamantyl etc.).

The term "$C_4$-$C_{10}$ cycloalkenyl" in the both cases, "$C_4$-$C_{10}$ cycloalkenyl" and "$C_4$-$C_{10}$ cycloalkenyl in the $C_4$-$C_{10}$ cycloalkenyloxy" means a cyclic alkenyl group having from 4 to 10 carbon atoms (e.g. bicyclo[4,3,0]nona-3-en-8-yl, 3-cyclopentenyl, 3-cyclohexenyl, 3-cycloheptenyl, tetrahydro-2-indanyl etc.).

The term "hydroxy $C_1-C_{12}$ alkyl" means a straight or branched alkyl group which has from one to 12 carbon atoms and which is substituted with a hydroxy group (e.g. hydroxymethyl, 2-hydroxyethyl, 3-hydroxypropyl, 4-hydroxybutyl, 5-hydroxypentyl, 6-hydroxyhexyl, 7-hydroxyheptyl, 8-hydroxyoctyl, 10-hydroxydecyl, 5-hydroxyhexyl, 4-hydroxypentyl, 5-hydroxy-1,1-dimethylpentyl, 5-hydroxy-2-methylpentyl, 5-hydroxy-1-methylpentyl, 6-hydroxy-2-methylhexyl etc.).

The term "$C_3-C_{10}$ heterocyclic group" means a monocyclic or dicyclic group having from 3 to 10 carbon atoms and at least one of hetero atoms selected from nitrogen atoms, sulfur atoms, and oxygen atoms (e.g. piperidine, morpholine, pyrrolidine, piperazine, tetrahydrofuran, tetrahydrothiophene, furan, thiophene, imidazole, pyridine, oxazole, isooxazole, pyrrole, pyrazole, pyrimidine, indole, benzofuran, purine, benzothiophene, quinoline, pyrrolidone, dihydrothiophene, dihydrobenzofuran, 1,4-benzodioxane, etc.).

The term "$C_1-C_7$ alkylene" means a straight or branched alkylene chain having from one to 7 carbon atoms (e.g. methylene, ethylene, trimethylene, tetramethylene, pentamethylene, hexamethylene, heptamethylene, methylmethylene, dimethylmethylene, 1,1-dimethylethylene, 2-methyltetramethylene, 1-methylpentamethylene, 2-methylhexamethylene, 1-ethylethylene, 2-ethylethylene, 2-ethyltrimethylene, etc.).

The term "5 to 7 membered saturated heterocyclic group" includes piperidine, morpholine, pyrrolidine, homopiperidine, piperazine, N-($C_1$-$C_4$) alkylpiperazine, etc.

The term "$C_3$-$C_7$ cycloalkyl" means a cyclic alkyl group which is optionally substituted with a $C_1$-$C_4$ alkyl group and which has from 3 to 7 carbon atoms (e.g. cyclopropyl, cyclobutyl, cyclopentyl, cyclohexyl, cycloheptyl, 3-ethylcyclopentyl, 4-methylcyclohexyl, etc.).

The term "esterified carboxyl group" includes $C_1$-$C_4$ alkoxycarbonyl, aryloxycarbonyl (e.g. phenoxycarbonyl, naphthoxycarbonyl), aralkyloxycarbonyl (e.g. benzyloxycarbonyl, phenethyloxycarbonyl), ($C_1$-$C_4$ alkoxy)-methoxycarbonyl, ($C_2$-$C_5$ alkanoyloxy)methoxycarbonyl (e.g. acetoxymethoxycarbonyl), ($C_3$-$C_7$ cycloalkyloxy)carbonyl, arylcarbonylmethoxycarbonyl and (hydroxy $C_1$-$C_4$ alkoxy)-carbonyl.

In 1979 B. Sammuelsson reported isolation and biological effects of leucotrienes (Sammuelsson, B. et al. (1980): In: Advances in Prostaglandin and Thronboxane Research, Vol. 6, edited by B. Sammuelsson, R. Ramwell, and R. Paaletti, P. I. Raven Press, New York).

Since then, a tremendous amount of research in synthetic organic chemistry and pharmacology of leucotriene $A_4$, $B_4$, $C_4$, $D_4$, etc. has been performed.

Leucotrienes induce an increase in capillary

permeability and cause smooth muscle contraction. Furthermore leucotriene $B_4$, one of leucotrienes which is shown below, has also different pharmacological properties from the others. It is chemotactic for macropharges and neutrophils at concentrations of ~1 ng/ml (greater than any other known lipid chemotactic factor). It is detected in the synovia of patients with rheumatoid arthritis or gouty arthritis, and in the sputum of obstructive airways diseases. They suggest that it is a primary mediator of inflammatory and allergic states. So, we have been making research to find novel types of drugs for use against inflammatory or allergic states.

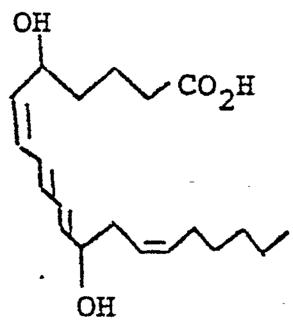

Leucotriene $B_4$

As the result of studies, it has now been found that the novel unsaturated fatty acids [I] of the present invention and their non-toxic pharmaceutically acceptable salts, have potent anti-leucotriene $B_4$ action which include anti-chemotactic activity, etc. This action may render these compounds very useful as the drugs for such inflammations or allergies.

Accordingly, a basic object of the present invention is to provide the novel unsaturated fatty acids [I] having excellent pharmacological activities.

Another object of the present invention is to provide processes for producing those compounds [I]. A further object of the present invention is to provide a pharmaceutical composition containing a compound of the formula [I]. These and other objects will be apparent to those skilled in the art to which the present invention pertains from the foregoing and subsequent descriptions.

The novel unsaturated fatty acids [I] of the invention can be prepared from the compound of the formula [II]:

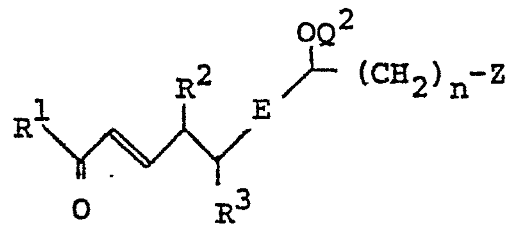

/II/

wherein $Q^2$ is a hydrogen atom or an acyl group, Z is an esterified carboxyl group or a group of the formula:

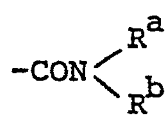

(wherein $R^a$ and $R^b$ are as defined above), and $R^1$, $R^2$, $R^3$, E and n are as defined above, by reacting the latter with a reducing agent, optionally followed by deprotection of a protected hydroxyl group, hydrolysis of an ester group, esterification of a carboxyl group, amidation of a free or esterified carboxyl group, and/or transesterification.

In the significances as used above, the term "an acyl group" means a $C_1$-$C_4$ alkanoyl group, a benzoyl

group or a substituted benzoyl group (e.g. acetyl, propionyl, benzoyl, p-phenyl benzoyl, etc.).

A part of the unsaturated fatty acids [I] can be also prepared by the following two methods.

The unsaturated fatty acids of the formula [III]:

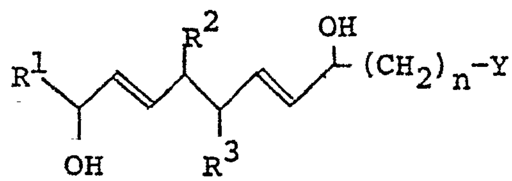

[III]

wherein $R^1$, $R^2$, $R^3$, n and Y are each as defined above, which are part of the unsaturated fatty acids [I], can be obtained from a carbonyl compound of the formula [IV]:

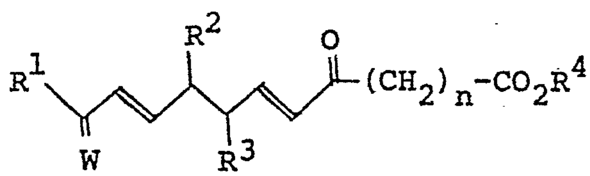

[IV]

wherein $R^1$, $R^2$, $R^3$, and n are each as defined above, $R^4$ is a $C_1$-$C_4$ alkyl group, and W is an oxygen atom or a group of the formula:

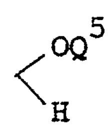

(wherein $Q^5$ is an acyl group or a substituted alkoxymethyl group which forms acetal with the adjoining oxygen atom), by reacting the latter with a reducing agent, optionally followed by deprotection of a protected hydroxyl group, hydrolysis of an ester group, esterification of a carboxyl group, amidation of a free or esterified carboxyl group, and/or transesterification. In the significances as used above, the term "a substituted alkoxymethyl group" means a protective group of a hydroxyl group such as tetrahydropyranyl, tetrahydrofuranyl, 1-ethoxyethyl, etc.

The unsaturated fatty acids [V]:

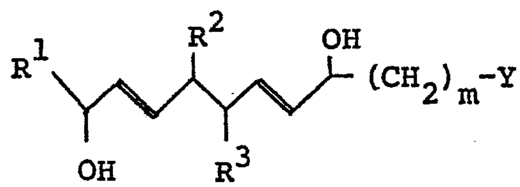

[V]

wherein m is 3 or 4, $R^1$, $R^2$, $R^3$ and Y are each as defined above, which are part of the unsaturated fatty acids [I], can be obtained from aldehydes of the formula [VI]:

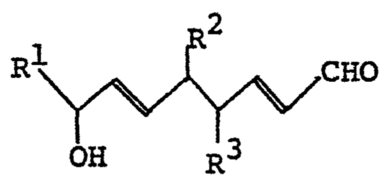

[VI]

wherein $R^1$, $R^2$ and $R^3$ are each as defined above, by reacting the latter with an organometallic compound of the formula [VII]:

$$M^1-(CH_2)m-C(OR^5)_3 \qquad [VII]$$

wherein $M^1$ is a lithium atom or a group of the formula: MgX (X is a chlorine atom, a bromine atom or an iodine atom), $R^5$ is a $C_1$-$C_4$ alkyl group, and m is 3 or 4, followed by transformation of an orthoester group to an ester group, and optionally followed by hydrolysis of an ester group,

esterification of a carboxyl group, amidation of a free or esterified carboxyl group and/or transesterification.

The sequence of the steps from the carbonyl compounds [II], [IV] and [VI] to the respective unsaturated fatty acids [I], [III] and [V] may be represented by the following scheme.

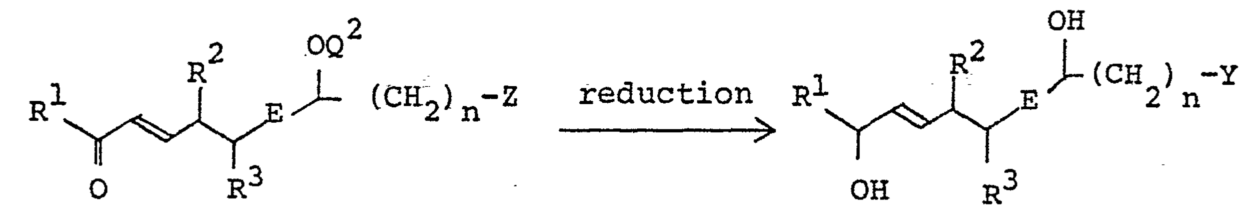


[II] [I]

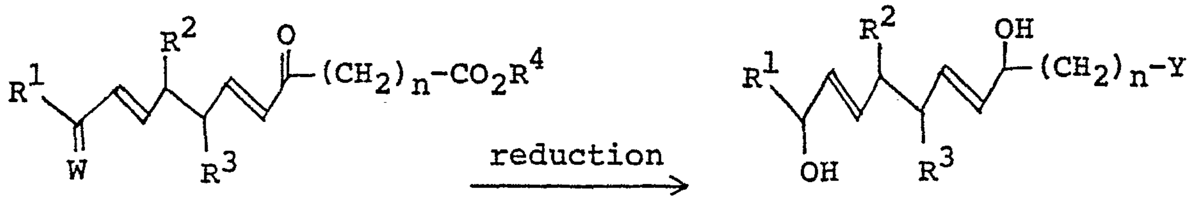


[IV] [III]

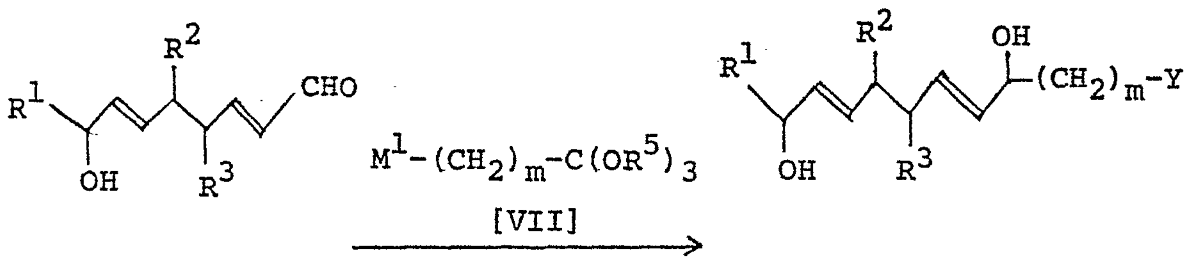


[VI] [V]

Scheme A

STEP 1.

Reduction of the carbonyl compounds [II] and [IV] to the respective unsaturated fatty acids [I] and [III].

The carbonyl compound [II] or [IV] can be converted into the corresponding alcohol by reacting the former with a reducing agent in an inert solvent (e.g. THF, ether, dimethoxyethane, pentane, hexane, benzene, toluene, methanol, ethanol, etc.) at a temperature in the range from -78°C to room temperature.

As the reducing agent, there may be used for example trialkylborohydride (e.g. lithium triisobutylborohydride), bis(2,4,6-tri-t-butylphenoxy) aluminum hydride, sodium borohydride, zinc borohydride, diisobutyl aluminum hydride, ethoxy 1,1'-binaphthyl-2,2'-dioxyaluminum lithium hydride, sodium trimethoxyaluminum hydride, sodium trimethoxyborohydride, etc.

The deprotection of a protected hydroxyl group can be carried out by the conventional procedure [Protective Group in Organic Chemistry. Edited by J. F. W. McOmie (1973) 95-143].

STEP 2.

Reaction of the aldehyde [VI] with an organometallic compound [VII]

The aldehyde [VI] can be converted into the corresponding intermediate ortho-ester by reacting of the former with an organometallic compound [VII] in an

inert solvent (e.g. ether, THF) at a temperature in the range from -78°C to room temperature. The organometallic compound [VII] can be prepared by reacting a halide of the formula [VIII]:

$$X-(CH_2)_m-C(OR^5)_3 \qquad [XIII]$$

wherein X, m and $R^5$ are each as defined above, with lithium or magnesium. If magnesium is selected in this reaction, active magnesium is preferably used [J. Org. Chem., 46, 4323 (1981)]. The halide [VIII] can be prepared by the method of the literature [R. H. DeWolfe, Synthesis, 1974, 153].

The transformation of an orthoester group to an ester group can be carried out by treating an orthoester compound with an acid (e.g. hydrogen chloride, silica gel, etc.) in an inert solvent (e.g. methanol, THF, ethyl acetate, etc.) at a temperature in the range from -30°C to room temperature.

The steps of hydrolysis of an esterified carboxyl group, esterification of a carboxyl group, amidation of a free or esterified carboxyl group and transesterification may be represented by the following scheme.

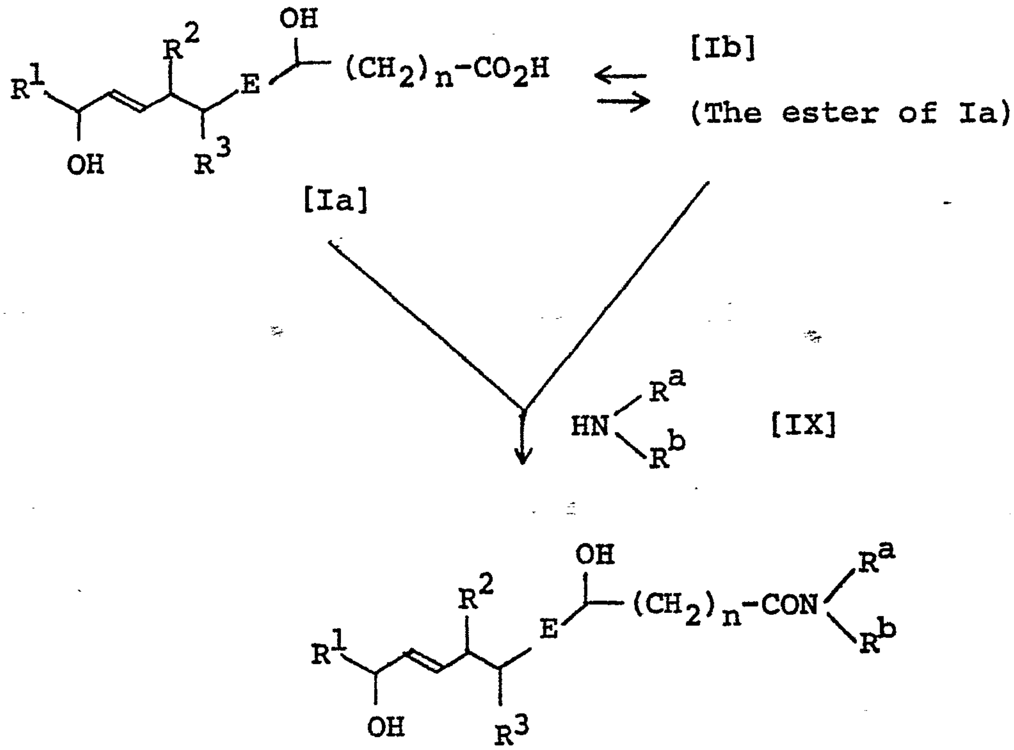
[Ib]
(The ester of Ia)
[Ia]
[IX]

[Ic]

Scheme B

Amidation of an esterified carboxyl group can be carried out by treating an ester compound [Ib] with an amine [IX] in an inert solvent (e.g. DMF, methanol, ethanol, THF, water) at a temperature in the range from room temperature to the boiling temperature of the solvent.

Amidation of a carboxyl compound [Ia], hydrolysis of an ester compound [Ib], esterification of a carboxyl compound, and transesterification of an ester compound [Ib] can be carried out by conventional procedures.

The carbonyl compound [II] used as an intermediate in the present invention can be prepared by the two methods shown in the following scheme.

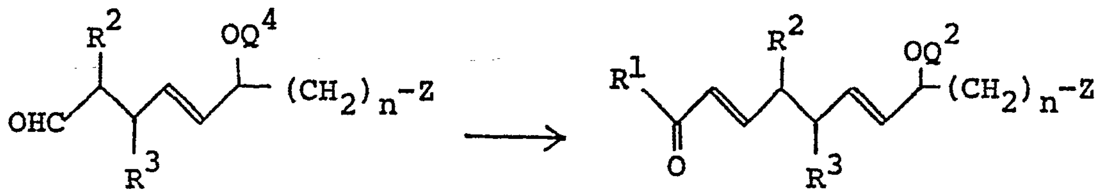

[X] [IIa]

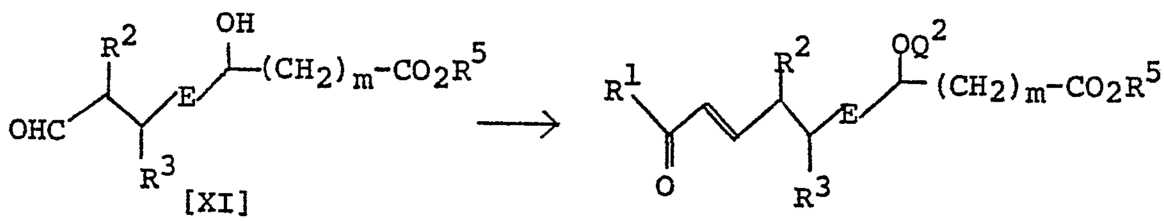

[XI] [IIb]

Scheme C

In the formulas illustrated in the scheme C, $R^1$, $R^2$, $R^3$, $R^5$, E, n, m, $Q^2$ and Z are each as defined above, and $Q^4$ is an acyl group.

Wittig reaction of the aldehyde [X] into the carbonyl compound [IIa] which is part of [II], can be accomplished by reacting the former with a compound of the formula [XII] or [XIII]:

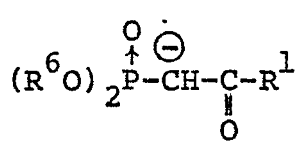

[XII]

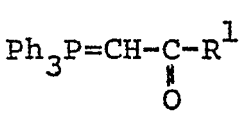

[XIII]

wherein $R^6$ is a $C_1$-$C_4$ alkyl group, Ph is a phenyl group, and $R^1$ is as defined above, in an inert solvent (e.g. dioxane, ether, THF, dimethoxyethane, benzene, toluene, n-hexane, DMSO) at a temperature in the range from -30°C to the boiling temperature of the solvent, optionally followed by deprotection of a protected hydroxyl group.

Wittig Reaction of the aldehyde [XI] into the carbonyl compound [IIb] which is part of [II], can be carried out by the same method as used in the synthesis of the carbonyl compound [IIa] from the aldehyde [X], optionally followed by protection of a hydroxyl group.

The aldehyde [X] can be prepared from a diol compound [XIV] by the process shown in the following scheme.

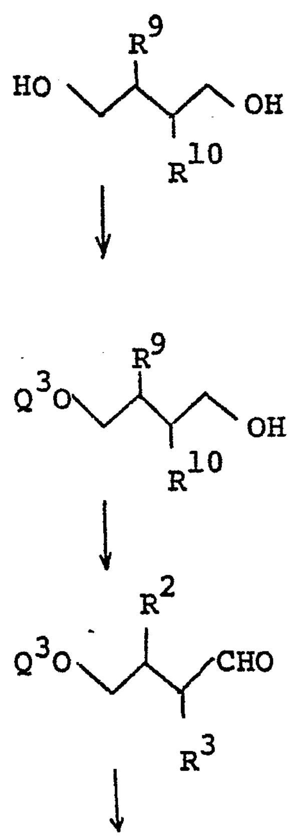

[XIV]

[XV]

[XVI]

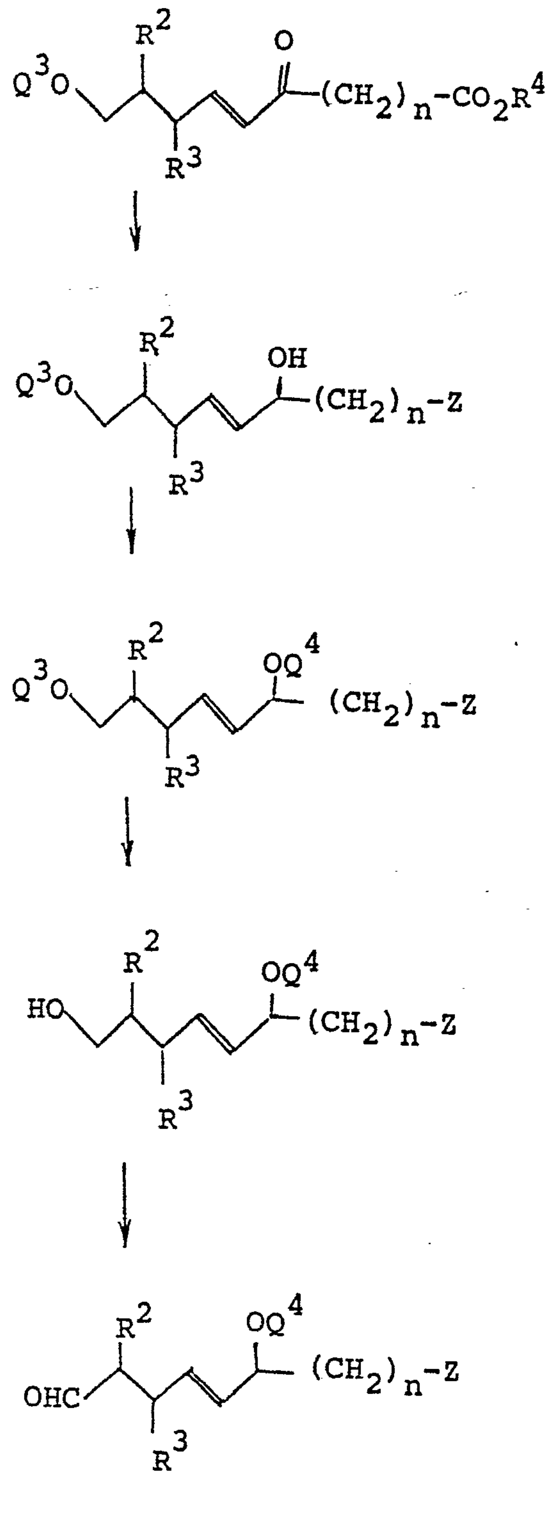

[XVII]

[XVIII]

[XIX]

[XX]

[X]

Scheme D

In the formulas illustrated in the scheme D, $Q^3$ is a substituted alkoxymethyl group which forms an acetal with the adjoining oxygen atom, $R^9$ and $R^{10}$ are hydrogen atoms or, when taken together, mean a single linkage to form a *cis* or *trans* double bond between the adjacent carbon atoms, and $R^2$, $R^3$, $R^4$, n, Z and $Q^4$ are each as defined above.

The detailed explanation of scheme D is as follows:

Mono protection of the diol [XIV] into the compound [XV] can be accomplished by treating the former with a reagent which makes an acetal by reacting itself with a hydroxy group (e.g. 3,4-dihydro-2H-pyran, 5,6-dihydro-4-methoxy-2H-pyran, ethyl vinyl ether, etc.) in the presence of a catalytic amount of an acid (e.g. sulfuric acid, p-toluenesulfonic acid, pyridinium 4-toluenesulfonate) in an inert solvent (e.g. methylene chloride, THF) at a temperature in the range from 0°C to 50°C.

Oxidation of the compound [XV] to the aldehyde [XVI] can be carried out by reacting the former with a chromium compound (e.g. pyridinium chlorochromate, chromium trioxide-pyridine complex (Collins reagent)) in an inert solvent (e.g. methylene chloride, chloroform) at a temperature in the range from -20°C to 40°C, followed by isomerization of the *cis* double bond into the *trans* double bond by reacting it with a base, in case that $R^9$ and $R^{10}$ mean a single linkage to form a *cis* double bond in the

formula [XV].

In case that $R^9$ and $R^{10}$ mean a single linkage to form a *cis* or *trans* double bond in the formula [XV], oxidation of the compound [XV] into the aldehyde [XVI] can be also carried out by reacting the former with active manganese dioxide in an inert solvent (e.g. methylene chloride, chloroform, benzene) at a temperature in the range from 0°C to the boiling temperature of the solvent, followed by isomerization of the *cis* double bond into the *trans* double bond by reacting it with a base, in case that $R^9$ and $R^{10}$ mean a single linkage to form a *cis* double bond in the formula [XV].

The ester [XVII] can be obtained from the aldehyde [XVI] by reacting the latter with the compound of the formula [XXI] or [XXII]:

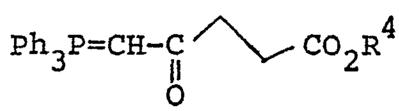

[XXI]

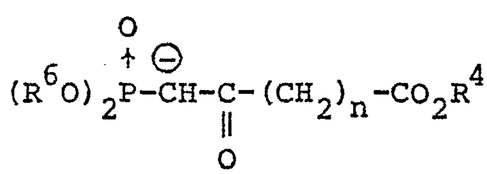

[XXII]

wherein Ph, $R^4$, $R^6$ and n are each as defined above, in an inert solvent (e.g. N,N-dimethylformamide, THF, ether, dimethoxyethane, benzene, toluene, n-hexane, DMSO) at a temperature in the range from -30°C to the boiling temperature of the solvent.

The ester [XVII] can be converted into the compound [XVIII] by the same procedure as used in the synthesis of the unsaturated fatty acids [I] from the carbonyl compound [II].

The compound [XVIII] can be converted into the compound [XIX] by treating the former with an acyl halide or an acid anhydride (e.g. acetic anhydride, acetyl chloride, benzoyl chloride, propionyl chloride) in the presence of a base (e.g. pyridine, triethylamine, 4-N,N-dimethylaminopyridine) in an inert solvent (e.g. methylene chloride, THF) at a temperature in the range from 0°C to 50°C.

The compound [XIX] can be converted into the compound [XX] by the conventional procedure.

The compound [XX] can be converted into the aldehyde [X] by the same procedure as used in the synthesis of the aldehyde [XVI] from the compound [XV].

The aldehyde [XI] can be prepared from the aldehyde [XXIII] by the process shown in the following scheme.

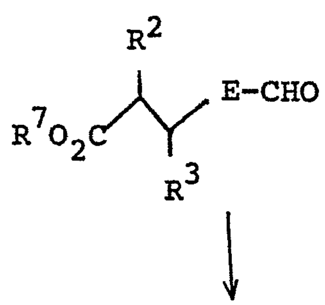

[XXIII]

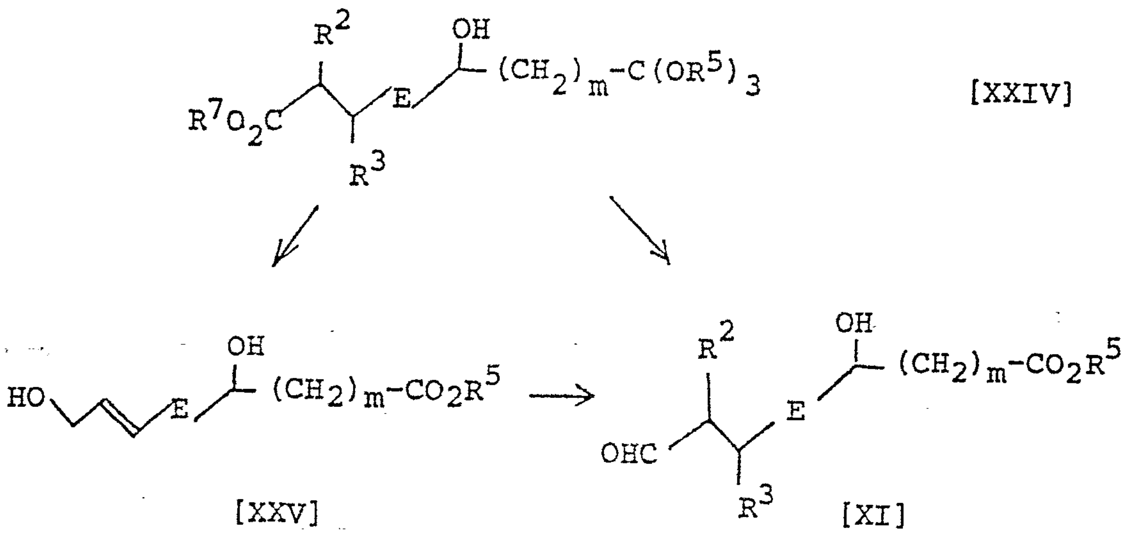

[XXIV]

[XXV] [XI]

Scheme E

In the formulas illustrated in the scheme E, $R^7$ is a $C_1$-$C_4$ alkyl group, and $R^2$, $R^3$, E, m and $R^5$ are each as defined above.

Detailed explanation of the scheme E:

The ester [XXIV] can be obtained from the aldehyde [XXIII] by the same procedure as used in the synthesis of the unsaturated fatty acids [V] from the aldehyde [VI].

Partial reduction of the ester [XXIV] to the aldehyde [XI] can be carried out by reacting the former with a reducing agent (e.g. diisobutylaluminum hydride) in an inert solvent (e.g. n-hexane, toluene, tetrahydrofuran) at a temperature in the range from -78°C to -30°C, followed by treating the resulting compound with an acid (e.g. hydrogen chloride, silica gel) in an inert solvent (e.g. methanol, THF, ethyl acetate)

at a temperature in the range from -30°C to room temperature.

The ester of the formula [XXIVa]:

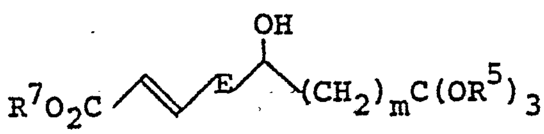

[XXIVa]

wherein $R^7$, E, m and $R^5$ are each as defined above, which is part of the ester [XXIV] can be converted into the alcohol [XXV] by reacting the former with a reducing agent (e.g. sodium trimethoxyaluminum hydride, lithium tri-s-butylborohydride, diisobutylaluminum hydride) in an inert solvent (e.g. THF, ether) at a temperature in the range from -78°C to room temperature, followed by treating the resulting compound with an acid (e.g. hydrogen chloride, silica gel) in an inert solvent (e.g. methanol, THF, ethyl acetate) at a temperature in the range from -30°C to room temperature.

The alcohol [XXV] can be also converted into the aldehyde of the formula [XIa]:

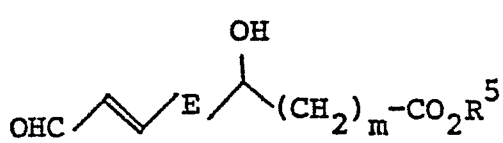

[XIa]

wherein E, m and $R^5$ are as defined above, by reacting the former with active manganese dioxide in an inert solvent (e.g. methylene chloride, chloroform, benzene, ethyl acetate) at a temperature in the range from 0°C to the boiling temperature of the solvent.

In case that E is a cis vinylene group in the formula [XXV] or [XIa], isomerization of a *cis* olefin into a *trans* olefin in the compound [XXV] or [XIa] can be easily accomplished by treating with an acid (e.g. hydrogen chloride).

The carbonyl compound [IV] used as an intermediate in the present invention can be prepared by the following scheme.

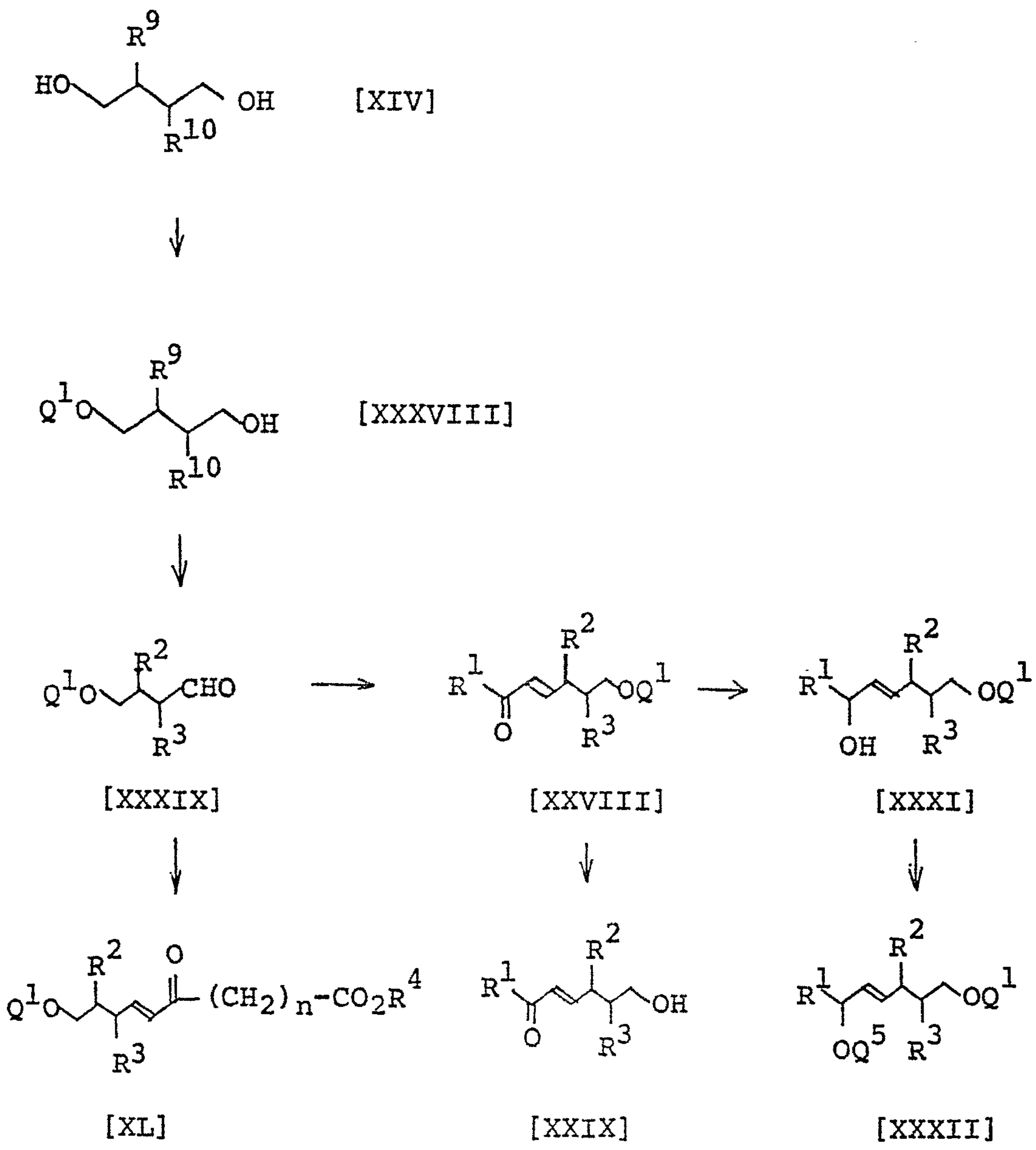

[XIV]

[XXXVIII]

[XXXIX] [XXVIII] [XXXI]

[XL] [XXIX] [XXXII]

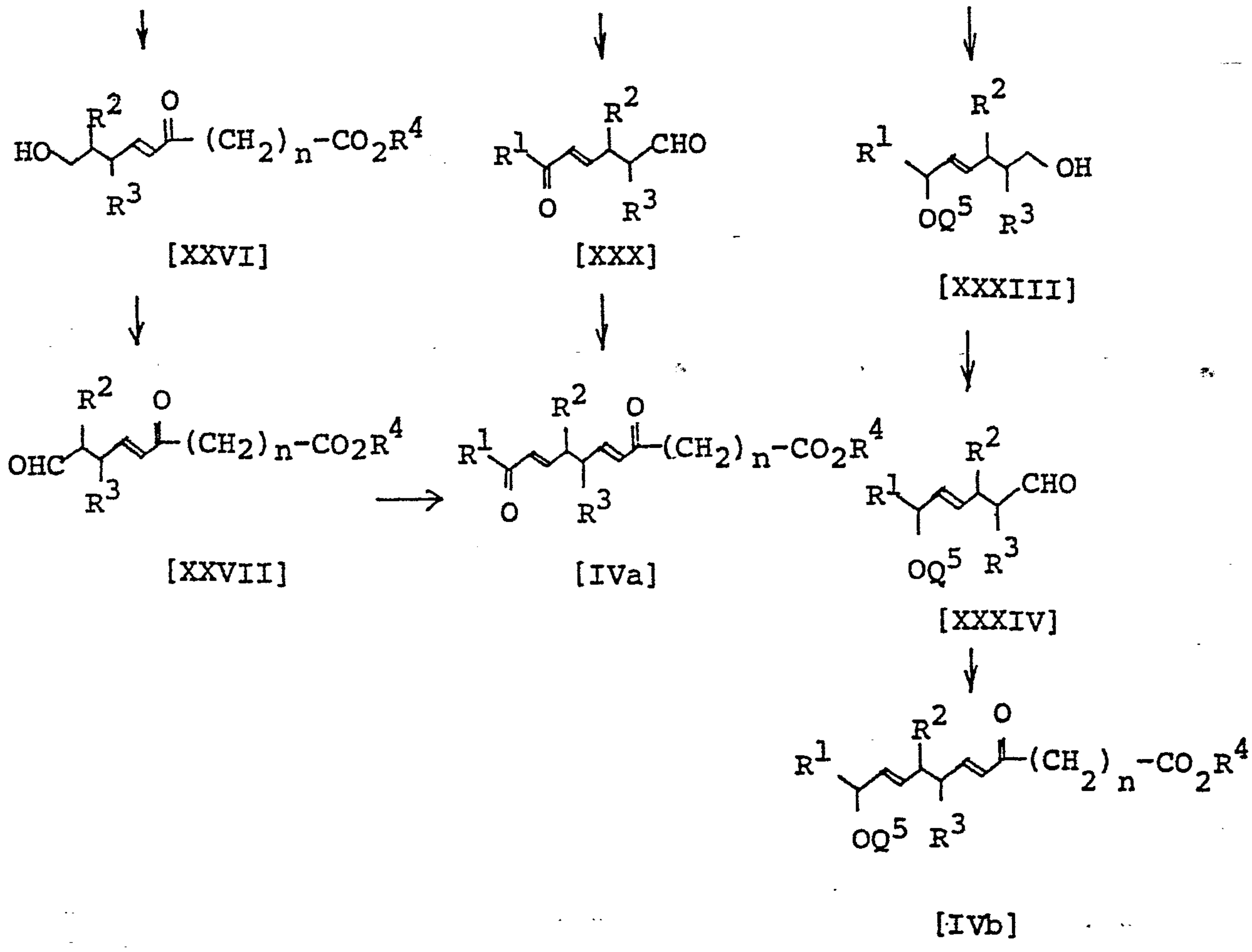

[XXVI] [XXX] [XXXIII]

[XXVII] [IVa] [XXXIV]

[IVb]

Scheme F

In the formulas illustrated in the scheme F, $R^1$, $R^2$, $R^3$, $R^4$, $R^9$, $R^{10}$, $Q^5$ and n are each as defined above, and $Q^1$ is an acyl group, when $Q^5$ is a substituted alkoxymethyl group which forms acetal with the adjoining oxygen atom, or $Q^1$ is a substituted alkoxymethyl group which forms acetal with the adjoining oxygen atom, when $Q^5$ is an acyl group.

Detailed explanation of the scheme F:

Mono protection of the diol [XIV] to form the compound [XXXVIII] can be accomplished by the same

procedure as used in the synthesis of the compound [XV] starting from the diol [XIV] or in the synthesis of the compound [XIX] from the compound [XVIII], or by continuous extraction from the aqueous solution of the diol [XIV] containing acetic acid in the presence of an acid (e.g. sulfuric acid) with a non-polar solvent (e.g. benzene, hexane) at a temperature in the range from 0°C to 50°C.

The compound [XXXVIII] can be converted into the aldehyde [XXXIX] by the same procedure as used in the synthesis of the aldehyde [XVI] from the compound [XV].

The aldehyde [XXXIX] can be converted into the ester [XL] by the same procedure as used in the synthesis of the compound [XVII] from the aldehyde [XVI].

The ester [XL] can be converted into the alcohol [XXVI] by the conventional procedure.

The alcohol [XXVI] can be converted into the aldehyde [XXVII] by the same procedure as used in the synthesis of the aldehyde [XVI] from the alcohol [XV].

The aldehyde [XXVII] can be converted into the carbonyl compound [IVa] which is part of the carbonyl compound [IV], by the same procedure as used in the synthesis of the carbonyl compound [IIa] from the aldehyde [X].

The carbonyl compound [IVa] can be also prepared in the following way.

The aldehyde [XXXIX] can be converted into the compound [XXVIII] by the same procedure as used in the synthesis of the carbonyl compound [IIa] from the

aldehyde [X].

The compound [XXVIII] can be converted into the alcohol [XXIX] by the conventional procedure.

The alcohol [XXIX] can be converted into the aldehyde [XXX] by the same procedure as used in the synthesis of the aldehyde [XVI] from the alcohol [XV].

The aldehyde [XXX] can be converted into the carbonyl compound [IVa] by the same procedure as used in the synthesis of the ester [XVII] from the aldehyde [XVI].

The carbonyl compound [IVb], which is part of the carbonyl compound [IV], can be prepared in the following way.

The reduction of the compound [XXVIII] into the alcohol [XXXI] can be accomplished by the same procedure as used in the synthesis of the unsaturated fatty acid [I] from the carbonyl compound [II].

The compound [XXXI] can be converted into the compound [XXXII] by the same procedure as used in the synthesis of the compound [XV] from the diol [XIV], when $Q^1$ is an acyl group in the formula [XXXI], or by the same procedure as used in the synthesis of the compound [XIX] from the compound [XVIII], when $Q^1$ is a substituted alkoxymethyl group.

The compound [XXXII] can be converted into the compound [XXXIII] by a conventional procedure.

The compound [XXXIII] can be converted into the aldehyde [XXXIV] by the same procedure as used in

the synthesis of the aldehyde [XVI] from the alcohol [XV].

The aldehyde [XXXIV] can be converted into the carbonyl compound [IVb] by the same procedure as used in the synthesis of the ester [XVII] from the aldehyde [XVI].

The aldehyde [VI] used as an intermediate in the present invention can be prepared by the following scheme.

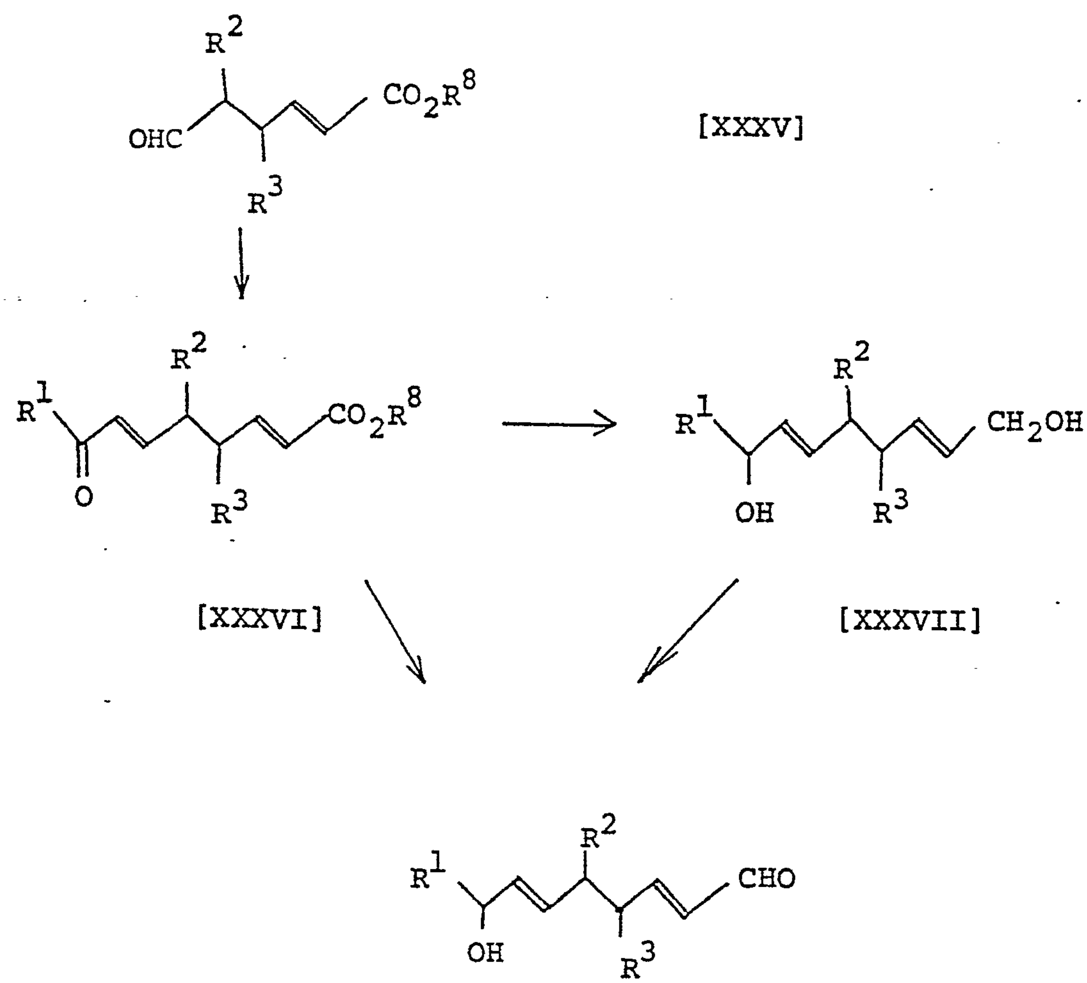

[XXXV]

[XXXVI]

[XXXVII]

[VI]

Scheme G

In the formulas illustrated in the scheme G, $R^8$ is a $C_1$-$C_4$ alkyl group, $R^1$, $R^2$ and $R^3$ are each as defined above.

Detailed explanation of the scheme G:

Wittig reaction of the aldehyde [XXXV] into the compound [XXXVI] can be carried out by the same procedure as used in the synthesis of the carbonyl compound [IIa] from the aldehyde [X].

Partial reduction of the ester [XXXVI] into the aldehyde [VI] can be accomplished by the same procedure as used in the synthesis of the aldehyde [XI] from the ester [XXIV].

Reduction of the ester [XXXVI] into the alcohol [XXXVII] can be carried out by the same procedure as used in the synthesis of the alcohol [XXV] from the ester [XXIVa].

The alcohol [XXXVII] can be converted into the aldehyde [VI] by the same procedure as used in the synthesis of the aldehyde [XIa] from the alcohol [XXV].

According to the present invention, the two stereoisomers of the formulas:

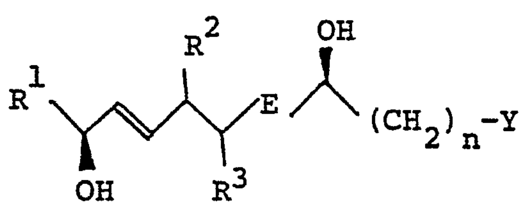

[A]

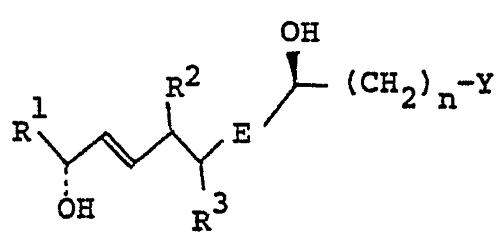

[B]

which include their corresponding enantiomers, can be prepared.

In general, the unsaturated fatty acid [I] can be obtained as a mixture of these stereoisomers, which can be easily separated by a conventional method (e.g. HPLC) with high purity. If necessary, a *cis* or *trans* isomer of a double bond in the unsaturated fatty acid [I] can be separated by a conventional method (e.g. HPLC).

Furthermore, the unsaturated fatty acid [I] can be separated into optical isomers by a conventional method.

Among the unsaturated fatty acids [I] thus obtained, the compound [Ia] can be converted to its pharmaceutically acceptable salt form. The pharmaceutically acceptable salts of these unsaturated fatty acids are those with pharmaceutically acceptable metal cations such as sodium, potassium, magnesium and calcium, ammonium or amine cations.

The preparation of pharmaceutical compositions can be carried out by a conventional method. For example, the unsaturated fatty acids [I] may be mixed with carriers, diluents, lubricants, fillers and/or binders such as lactose, sucrose, calcium phosphate, starch, talcum, casein, magnesium stearate, methyl cellulose, polyglycols, tragacanth and the like, sometimes together with stabilizers and emulsifying agents. The resulting mixture may be processed in a usual manner to tablets,

capsules, pills, ampoules and the like.

In a clinical practice, the unsaturated fatty acids [I] can be administered orally, intranasally, subcutaneously, intravenously, intramuscularly, intradermally or the like.

The daily dosage may vary depending upon the administration route and the usual oral dosage of the active ingreident is between about 0.1 mg and about 100 mg daily for human beings.

Specific examples of the unsaturated fatty acid [I] are as follows:

° (6Z,8E,10E)-5,12-dihydroxy-nonadeca-6,8,10-trienoic acid

° (6Z,8E,10E,15Z)-methyl 5,12-dihydroxy-eicosa-6,8,10,15-tetraenoate

° (6Z,8E,10E)-N,N-dimethyl-5,12-dihydroxy-eicosa-6,8,10-triene-14-ynamide

° (6Z,8E,10E)-methyl 5,12-dihydroxy-12-cyclohexyl-dodeca-6,8,10-trienoate

° (6Z,10E)-5,12,20-trihydroxy-eicosa-6,10-dienamide

° (6Z,8E,10E)-5,12-dihydroxy-12-(m-chlorophenyl)-dodeca-6,8,10-trienoic acid

° (6Z,8E,10E)-methyl 5,12-dihydroxy-12-(p-trifluoromethylphenyl)-dodeca-6,8,10-trienoate

° (6Z,10E)-5,12-dihydroxy-14-phenyl-tetradeca-6,10-dienoic acid

° (6E,8E,10E)-5,12-dihydroxy-13-cyclohexyl-trideca-6,8,10-trienoic acid

- (6Z,8E,10E)-5,12-dihydroxy-13-phenoxy-trideca-6,8,10-trienoic acid
- (6Z,10E)-5,12-dihydroxy-14-ethoxy-tetradeca-6,10-dienoic acid
- (6Z,10E)-N,N-dimethyl-5,12-dihydroxy-14-(p-methylphenyl)-tetradeca-6,10-dienamide
- (7Z,9E,11E)-methyl 6,13-dihydroxy-13-cyclopentyl-trideca-7,9,11-trienoate
- (7Z,11E)-6,13-dihydroxy-19-dimethylamino-nonadeca-7,11-dienamide
- (7Z,9E,11E)-methyl 6,13-dihydroxy-14-(m-chlorophenoxy)-tetradeca-7,9,11-trienoate
- (7E,9E,11E)-N,N-diethyl-6,13-dihydroxy-15-(p-dimethylaminophenyl)-pentadeca-7,9,11-trienamide
- (6Z,10E)-N,N-tetramethylene-5,12-dihydroxy-12-(p-hydroxyphenyl)-dodeca-6,10-dienamide
- (6Z,10E)-methyl 5,12-dihydroxy-12-(2'-pyridyl)-dodeca-6,10-dienoate
- (6Z,10E)-methyl 5,12-dihydroxy-14-(2'-furyl)-tetradeca-6,10-dienoate
- (7Z,11E)-N-(n-butyl)-6,13-dihydroxy-14-(1'-adamantyl)-tetradeca-7,11-dienamide
- (5E,7E,9E)-4,11-dihydroxy-11-cyclopentyl-undeca-5,7,9-trienamide
- (5E,7E,9E)-N,N-dimethyl-4,11-dihydroxy-12-cyclohexyl-dodeca-5,7,9-trienamide
- (5E,7E,9E)-methyl 4,11-dihydroxy-nonadeca-5,7,9-trienoate

° (5E,9E,13Z)-N,N-tetramethylene-4,11-dihydroxy-eicosa-5,9,13-trienamide

° (5E,7E,9E)-N-(n-butyl)-4,11-dihydroxy-12-methyl-hexadeca-5,7,9-triene-14-ynamide

° (5E,7E,9E)-4,11-dihydroxy-13-ethoxy-trideca-5,7,9-trienoic acid

° (5E,7E,9E)-N-benzyl-4,11-dihydroxy-12-phenyl-dodeca-5,7,9-trienamide

° (5E,9E)-N-methyl-4,11-dihydroxy-12-(p-fluorophenoxy)-dodeca-5,9-dienamide

° (5E,7E,9E)-methyl 4,11-dihydroxy-13-(3',4'-dichlorophenyl)-trideca-5,7,9-trienoate

° (5E,7E,9E)-4,11-dihydroxy-14-(p-methylphenyl)-tetadeca-5,7,9-trienoic acid

° (5E,7E,9E)-N,N-tetramethylene-4,11-dihydroxy-13-(p-dimethylaminophenyl)-trideca-5,7,9-trienamide

° (5E,9E)-N,N-diethyl-4,11-dihydroxy-14-(m-trifluoromethylphenyl)-tetradeca-5,9-dienamide

° (5E,7E,9E)-N-phenyl-4,11-dihydroxy-13-(p-methoxyphenyl)-trideca-5,7,9-trienamide

° (5E,7E,9E)-4,11-dihydroxy-15-(3',4',5'-trimethoxyphenyl)-pentadeca-5,7,9-trienamide

° (5E,7E,9E)-N-ethyl-4,11-dihydroxy-15-dimethylamino-pentadeca-5,7,9-trienamide

° (5E,7E,9E)-N,N-dimethyl-4,11-dihydroxy-13-(m-hydroxyphenyl)-trideca-5,7,9-trienamide

° (5E,7E,9E)-ethyl 4,11-dihydroxy-11-phenyl-undeca-5,7,9-trienoate

° (5E,7E,9E)-N,N-tetramethylene-4,11-dihydroxy-13-(4'-methylthiophenyl)-trideca-5,7,9-trienamide

° (5E,9E)-N-cyclohexyl-4,11,17-trihydroxy-heptadeca-5,9-dienamide

° (5E,7E,9E)-N-ethyl-4,11-dihydroxy-13-(2'-pyridyl)-trideca-5,7,9-trienamide

Practical and preferred embodiments of the present invention are illustratively shown in the following referential examples and examples.

Referential Example 1

To a N,N-dimethylformamide solution (20 ml) of (2Z)-2-butene-1,4-diol (15 g), was added methylene chloride (200 ml), 3,4-dihydro-2H-pyran (15 g) and p-toluenesulfonic acid monohydrate (2 g) sequentially. The reaction mixture was stirred at room temperature for 6 hrs. and then washed with sodium bicarbonate aqueous solution, dried, concentrated and chromatographed on silica gel to give (2Z)-4-tetrahydropyranoxy-2-butene-1-ol (18 g).

NMR δ ($CDCl_3$)

1.6 (6H, br), 3.5 - 4.0 (2H, m), 4.67 (1H, S), 5.70 (1H, dt, 12/5 Hz) and 5.86 (1H, dt, 12/5 Hz)

Referential Example 2

Active manganese dioxide (50 g) was added to a

chloroform solution (100 ml) of (2Z)-4-tetrahydropyranoxy-2-butene-1-ol (11 g). The mixture was stirred at room temperature for 10 hrs. and filtrated through celite, concentrated and chromatographed on silica gel to give an oil. Then, to a tetrahydrofuran solution (75 g) of the above oil, was added 1,8-diazabicyclo[5,4,0]-7-undecene (1 g). The mixture was stirred at room temperature, and poured into water, and then extracted with ethyl acetate. The organic layer was dried, concentrated and chromatographed on silica gel to give (2E)-4-tetrahydropyranoxy-2-butenal (9 g).

NMR δ ($CDCl_3$; (2Z) isomer)

1.6 (6H, br), 3.5 - 4.0 (2H, m), 4.65 (3H, m), 6.02 (1H, ddt, 11, 7, and 2Hz), 6.66 (1H, dt, 11 and 5Hz), 10.14 (1H, d, 7Hz),

NMR δ ($CDCl_3$; (2E) isomer)

1.6 (6H, br), 3.5 - 4.0 (2H, m), 4.36 (2H, m), 4.66 (1H, brs), 6.37 (1H, ddt, 15, 7, and 2 Hz), 6.85 (1H, dt, 15 and 5Hz), 9.59 (1H, d, 7Hz)

Referential Example 3

Methyl 5-(triphenylphosphoranylidene)levulinate (35 g) was added to a N,N-dimethylformamide solution (100 ml) of (2E)-4-tetrahydropyranoxy-2-butenal (13 g), and the mixture was stirred at 50°C for 3 hrs. The reaction mixture was poured into water and extracted with ethyl acetate. The organic layer was dried, concentrated,

and chromatographed on silica gel to give (5E,7E)-methyl 9-tetrahydropyranoxy-4-oxo-nona-5,7-dienoate (9 g).

NMR δ ($CDCl_3$)

1.6 (6H, br), 2.63 (2H, t, 6Hz), 2.90 (2H, t, 6Hz), 3.68 (3H, S), 4.4 (2H, m), 4.67 (1H, brs), 6.0 - 7.7 (4H, m)

Referential Example 4

p-Toluenesulfonic acid monohydrate (200 mg) was added to a methanol solution (150 ml) of (5E,7E)-methyl 9-tetrahydropyranoxy-4-oxo-nona-5,7-dienoate (9 g), and the mixture was stirred at 50°C for 1 hr. Methanol was evaporated, and to the mixture was added sodium bicarbonate aqueous solution. Then the mixture was extracted with ethyl acetate. The extract was dried and concentrated to give an oil. Then, to a chloroform solution (100 ml) of the above oil, was added active manganese dioxide (20 g). The reaction mixture was stirred at room temperature for 10 hrs. and filtrated on celite, concentrated and chromatographed on silica gel to give (5E,7E)-methyl 8-formyl-4-oxo-octa-5,7-dienoate (6.4 g).

NMR δ ($CDCl_3$)

2.69 (2H, t, 6Hz), 3.01 (2H, t, 6Hz), 3.72 (3H, S), 6.5 (1H, m), 6.63 (1H, d, 15Hz), 7.26 (1H, dd, 15, 12Hz), 7.43 (1H, dd, 15, 12Hz), 9.79 (1H, d, 7Hz)

Referential Example 5

To a tetrahydrofuran suspension (100 ml) of sodium hydride (60% dispersion in mineral oil; 410 mg), was added dimethyl (2-oxo decyl)phosphonate (2.7 g), and then to the reaction mixture, was added a tetrahydrofuran solution (10 ml) of (5E,7E)-methyl 8-formyl-4-oxo-octa-5,7-dienoate (2 g) at 0°C. The reaction mixture was stirred at room temperature for 2 hrs. and poured into water, extracted with ethyl acetate. The extract was dried, concentrated and chromatographed on silica gel to give (5E,7E,9E)-methyl 4,11-dioxo-nonadeca-5,7,9-trienoate (800 mg).

NMR δ ($CDCl_3$)

0.88 (3H, brt), 2.6 (4H, m), 2.94 (2H, t, 6Hz), 3.68 (3H, S), 6.34 (2H, d, 15Hz), 6.6 - 7.4 (4H, m)

Referential Example 6

According to the same procedure as that of Referential Example 5, there were obtained the following compounds.

o (5E,7E,9E)-methyl 13,17-dimethyl-4,11-dioxo-octadeca-5,7,9,16-tetraenoate

NMR δ ($CDCl_3$)

0.92 (3H, d, 7Hz), 1.61 (3H, S), 1.68 (3H, S), 2.43 (2H, d, 7Hz), 2.64 (2H, t, 6Hz), 2.92 (2H, t, 6Hz), 3.71 (3H, S), 5.72 (1H, brt, 7Hz), 6.31 (2H, d, 15Hz), 6.61 (1H, dd, 14, 11Hz),

6.69 (1H, dd, 14, 11Hz), 7.18 (1H, dd, 15, 11Hz), 7.22 (1H, dd, 15, 11Hz)

o (5E,7E,9E)-methyl 13-(p-methoxyphenyl)-4,11-dioxo-trideca-5,7,9-trienoate

NMR δ ($CDCl_3$)

2.63 (2H, t, 6Hz), 2.87 (6H, m), 3.67 (3H, S), 3.76 (3H, S), 6.26 (1H, d, 15Hz), 6.29 (1H, d, 15Hz), 6.59 (1H, dd, 14, 11Hz), 6.66 (1H, dd, 14, 11Hz), 6.76 (2H, d, 8Hz), 7.08 (2H, d, 8Hz), 7.1 - 7.4 (2H, m)

o (5E,7E,9E)-methyl 13-(3',4'-dichlorophenyl)-4,11-dioxo-trideca-5,7,9-trienoate

NMR δ ($CDCl_3$)

2.66 (2H, t, 6Hz), 2.92 (6H, m), 3.69 (3H, S), 6.28 (1H, d, 15Hz), 6.31 (1H, d, 15Hz), 6.62 (1H, dd, 14, 11Hz), 6.71 (1H, dd, 14, 11Hz), 7.0 - 7.4 (5H, m)

o (5E,7E,9E)-methyl 13-(m-trifluoromethylphenyl)-4,11-dioxo-trideca-5,7,9-trienoate

NMR δ ($CDCl_3$)

2.65 (2H, t, 6Hz), 3.0 (6H, brm), 3.68 (3H, S), 6.28 (1H, d, 15Hz), 6.31 (1H, d, 15Hz), 6.60 (1H, dd, 14, 11Hz), 6.67 (1H, dd, 14, 11Hz), 7.1 - 7.6 (6H, m)

o (5E,7E,9E)-methyl 12-(p-fluorophenoxy)-4,11-dioxo-dodeca-5,7,9-trienoate

NMR δ ($CDCl_3$)

2.66 (2H, t, 6Hz), 2.93 (2H, t, 6Hz), 3.69 (3H, S), 4.66 (2H, S), 6.3 - 7.5 (10H, m)

o (5E,7E,9E)-methyl 14-(p-methylphenyl)-4,11-dioxo-tetradeca-5,7,9-trienoate

NMR δ ($CDCl_3$)

1.95 (2H, qui, 7Hz), 2.31 (3H, S), 2.93 (2H, t, 7Hz), 3.69 (3H, S), 6.27 (1H, d, 15Hz), 6.32 (1H, d, 15Hz), 6.60 (1H, dd, 15, 12Hz), 6.68 (1H, dd, 15, 12Hz), 7.08 (4H, S), 7.2 (2H, m)

o (5E,7E,9E)-methyl 12-cyclohexyl-4,11-dioxo-dodeca-5,7,9-trienoate

NMR δ ($CDCl_3$)

2.43 (2H, d, 6Hz), 2.66 (2H, t, 6Hz), 2.92 (2H, t, 6Hz), 3.69 (3H, S), 6.29 (1H, d, 15Hz), 6.31 (1H, d, 15Hz), 6.62 (1H, dd, 15, 12Hz), 6.71 (1H, dd, 15, 12Hz), 7.3 (2H, m)

o (5E,7E,9E)-methyl 13-(p-dimethylaminophenyl)-4,11-dioxo-trideca-5,7,9-trienoate

NMR δ ($CDCl_3$)

2.64 (2H, t, 6Hz), 2.86 (4H, S), 2.90 (6H, S), 3.68 (3H, S), 6.28 (1H, d, 15Hz), 6.30 (1H, d,

0183177

15Hz), 6.6 (2H, m), 6.66 (2H, d, 9Hz), 7.06 (2H, d, 9Hz), 7.2 (2H, m)

o (5E,7E,9E)-methyl 11-cyclopentyl-4,11-dioxo-undeca-5,7,9-trienoate

NMR δ ($CDCl_3$)

2.65 (2H, t, 6Hz), 2.93 (2H, t, 6Hz), 3.69 (3H, S), 6.33 (1H, d, 15Hz), 6.37 (1H, d, 15Hz), 6.65 (1H, dd, 15, 12Hz), 6.73 (1H, dd, 15, 12Hz), 7.2 (2H, m)

o (5E,7E,9E)-methyl 13-methyl-4,11-dioxo-heptadeca-5,7,9-trienoate

NMR δ ($CDCl_3$)

0.88 (3H, t, 6Hz), 0.91 (3H, d, 5Hz), 2.43 (2H, d, 7Hz), 2.67 (2H, t, 6Hz), 2.93 (2H, t, 6Hz), 3.69 (3H, S), 6.32 (2H, d, 15Hz), 6.63 (1H, dd, 15, 12Hz), 6.71 (1H, dd, 15, 12Hz), 7.2 (2H, m)

o (5E,7E,9E)-methyl 13-(o-methoxyphenyl)-4,11-dioxo-trideca-5,7,9-trienoate

NMR δ ($CDCl_3$)

2.65 (2H, t, 6Hz), 2.90 (2H, t, 6Hz), 2.91 (4H, brs), 3.68 (3H, S), 3.83 (3H, S), 6.27 (1H, d, 15Hz), 6.29 (1H, d, 15Hz), 6.6 - 7.4 (8H, m)

o (5E,7E,9E)-methyl 13-(m-methoxyphenyl)-4,11-dioxo-trideca-5,7,9-trienoate

NMR δ ($CDCl_3$)

2.65 (2H, t, 6Hz), 2.92 (2H, t, 6Hz), 2.93 (4H, brs), 3.69 (3H, S), 3.79 (3H, S), 6.30 (1H, d, 15Hz), 6.32 (1H, d, 15Hz), 6.6 - 7.4 (8H, m)

o (5E,7E,9E)-methyl 13-(3',4'-dimethoxyphenyl)-4,11-dioxo-trideca-5,7,9-trienoate

NMR δ ($CDCl_3$)

2.65 (2H, t, 6Hz), 2.90 (4H, brs), 2.92 (2H, t, 6Hz), 3.69 (3H, S), 3.86 (6H, S), 6.30 (1H, d, 15Hz), 6.32 (1H, d, 15Hz), 6.6 - 7.4 (7H, m)

o (5E,7E,9E)-methyl 13-(3',4',5'-trimethoxyphenyl)-4,11-dioxo-trideca-5,7,9-trienoate

NMR δ ($CDCl_3$)

2.66 (2H, t, 6Hz), 2.91 (4H, brs), 2.93 (2H, t, 6Hz), 3.69 (3H, S), 3.82 (3H, S) 3.84 (6H, S), 6.32 (2H, d, 15Hz), 6.41 (2H, S) 6.5 - 7.4 (4H, m)

Referential Example 7

According to the same procedure as that of Referential Example 1, from 1,4-butanediol was obtained 4-tetrahydropyranoxy-1-butanol.

NMR δ ($CDCl_3$)

1.7 (10H, br), 3.7 (6H, m), 4.59 (1H, brs)

Referential Example 8

To a methylene chloride suspension (200 ml) of pyridinium chlorochromate (13 g), was added a methylene chloride solution (20 ml) of 4-tetrahydropyranoxy-1-butanol (10 g) at 0°C. The reaction mixture was stirred at room temperature for 3 hrs., and diluted with n-hexane (200 ml), filtrated on celite and concentrated to give an oil. Then the above oil was dissolved in n-hexane (200 ml), filtrated on celite, concentrated to give an oil (10 g).

Then, according to the same procedure as Referential Example 3, from the above oil (10 g) was obtained (5E)-methyl 9-tetrahydropyranoxy-4-oxo-5-nonenoate (7 g).

NMR δ ($CDCl_3$)

1.6 (8H, br), 2.36 (2H, q, 7Hz), 2.63 (2H, t, 6Hz), 2.89 (2H, t, 6Hz), 3.69 (3H, S), 4.56 (1H, brs), 6.13 (1H, d, 15Hz), 6.93 (1H, dt, 15, 7Hz)

Referential Example 9

To a methanol solution (150 ml) of (5E)-methyl 9-tetrahydropyranoxy-4-oxo-5-nonenoate (7 g), was added p-toluenesulfonic acid monohydrate (200 mg). The reaction mixture was stirred at 50°C for 1 hr. Methanol

was evaporated, and to the mixture was added sodium bicarbonate aqueous solution. Then the mixture was extracted with ethyl acetate. The extract was dried and concentrated to give an oil. Then to a methylene chloride solution (80 ml) of pyridinium chlorochromate (4 g), was added a methylene chloride solution (10 ml) of the above oil at 0°C. The reaction mixture was stirred at room temperature for 3 hrs., and diluted with n-hexane (80 ml), filtrated on celite, concentrated to give an oil. Then the above oil was dissolved in n-hexane (80 ml), filtrated on celite, concentrated to give (5E)-methyl 8-formyl-4-oxo-5-octenoate (2 g).

NMR δ ($CDCl_3$)

2.7 (8H, m), 3.68 (3H, S), 6.13 (1H, d, 15Hz), 6.85 (1H, dt, 15, 7Hz), 9.83 (1H, brs)

Referential Example 10

According to the same procedure as that of Referential Example 5, from (5E)-methyl 8-formyl-4-oxo-5-octenoate was obtained (5E,9E)-methyl 4,11-dioxo-nonadeca-5,9-dienoate.

NMR δ ($CDCl_3$)

0.87 (3H, br), 1.4 (4H, m), 2.52 (2H, t, 7Hz), 2.63 (2H, t, 6Hz), 2.87 (2H, t, 6Hz), 3.70 (3H, S), 6.12 (1H, d, 15Hz), 6.15 (1H, d, 15Hz), 6.8 (2H, m)

Referential Example 11

To a tetrahydrofuran suspension (200 ml) of sodium hydride (60% dispersion in mineral oil; 1 g), was added dimethyl (5-methoxycarbonyl-2-oxo-pentyl)-phosphonate (6.1 g), and then to the mixture was added a tetrahydrofuran solution (20 ml) of (2E)-4-tetrahydropyranoxy-2-butenal (4.1 g) at 0°C. The reaction mixture was stirred at room temperature for 2 hrs. and poured into water, extracted with ethyl acetate. The extract was dried, concentrated and chromatographed on silica gel to give (6E,8E)-methyl 10-tetrahydropyranoxy-5-oxo-deca-6,8-dienoate (3.2 g).

NMR δ ($CDCl_3$)

1.96 (2H, qui, 7Hz), 2.39 (2H, t, 7Hz), 2.67 (2H, t, 7Hz), 3.71 (3H, S), 4.4 (2H, m), 4.68 (1H, br), 5.9 - 7.7 (4H, m)

Referential Example 12

According to the same procedure as that of Referential Example 4, from (6E,8E)-methyl 10-tetrahydropyranoxy-5-oxo-deca-6,8-dienoate was obtained (6E,8E)-methyl 9-formyl-5-oxo-nona-6,8-dienoate.

NMR δ ($CDCl_3$)

2.40 (2H, t, 7Hz), 2.73 (2H, t, 7Hz), 3.69 (3H, S), 6.5 (1H, m), 6.53 (1H, d, 15Hz), 7.20 (1H, dd, 15, 11Hz), 7.32 (1H, dd, 15, 11Hz), 9.76 (1H, d, 8Hz)

Referential Example 13

According to the same procedure as that of Referential Example 5, from (6E,8E)-methyl 9-formyl-5-oxo-nona-6,8-dienoate was obtained (6E,8E,10E)-methyl 13-(p-fluorophenoxy)-5,12-dioxo-trideca-6,8,10-trienoate.

NMR δ ($CDCl_3$)

1.96 (2H, qui, 7Hz), 2.39 (2H, t, 7Hz), 2.67 (2H, t, 7Hz), 3.67 (3H, S), 4.66 (2H, S), 6.3 - 7.4 (10H, m)

Referential Example 14

According to the same procedure as that of Referential Example 13, there were obtained the following compounds.

o (6E,8E,10E)-methyl 14-(m-trifluoromethylphenyl)-5,12-dioxo-tetradeca-6,8,10-trienoate

NMR δ ($CDCl_3$)

1.96 (2H, qui, 7Hz), 2.38 (2H, t, 7Hz), 2.66 (2H, t, 7Hz), 2.99 (4H, brs), 3.67 (3H, S), 6.29 (2H, d, 15Hz), 6.60 (1H, dd, 14, 11Hz), 6.68 (1H, dd, 14, 11Hz), 7.1 - 7.6 (6H, m)

o (6E,8E,10E,14Z)-methyl 5,12-dioxo-tricosa-6,8,10,14-tetraenoate

NMR δ ($CDCl_3$)

0.88 (3H, br), 2.4 - 2.8 (6H, m), 3.68 (3H, S), 5.34 (2H, t, 5Hz), 6.30 (2H, d, 15Hz), 6.62 (1H, dd, 14, 11Hz), 6.70 (1H, dd, 14, 11Hz),

7.1 - 7.4 (2H, m)

o (6E,8E,10E)-methyl 14-(3',4'-dichlorophenyl)-5,12-dioxo-tetradeca-6,8,10-trienoate

NMR δ ($CDCl_3$)

1.96 (2H, qui, 7Hz), 2.39 (2H, t, 7Hz), 2.70 (2H, t, 7Hz), 2.91 (4H, br), 3.67 (3H, S), 6.28 (2H, d, 15Hz), 6.61 (1H, dd, 14, 11Hz), 6.69 (1H, dd, 14, 11Hz), 7.0 - 7.5 (5H, m)

o (6E,8E,10E)-methyl 15-(p-methylphenyl)-5,12-dioxo-pentadeca-6,8,10-trienoate

NMR δ ($CDCl_3$)

2.0 (4H, m), 2.31 (3H, S), 3.68 (3H, S), 6.27 (2H, d, 15Hz), 6.59 (1H, dd, 15, 12Hz), 6.67 (1H, dd, 15, 12Hz), 7.08 (4H, S), 7.2 (2H, m)

Referential Example 15

(6Z,8E)-Methyl 5,10-dihydroxy-deca-6,8-dienoate was obtained by the following three methods.

1) To a tetrahydrofuran suspension (50 ml) of magnesium (6.3 g) was added a tetrahydrofuran solution (10 ml) of trimethyl ortho-4-bromobutyrate (20 g) dropwise at about 25°C. Then, to a tetrahydrofuran solution (400 ml) of (2E,4Z)-ethyl 5-formyl-penta-2,4-dienoate (9.1 g) was added the above reaction mixture dropwise at about -50°C. The mixture was warmed gradually to room

temperature, and poured into water, extracted with ether. The extract was dried and concentrated to give an oil.

To a tetrahydrofuran solution (400 ml) of the above oil, was added diisobutylaluminum hydride (1.5M solution in toluene; 100 ml) at about -50°C. The mixture was warmed gradually to room temperature. Then, to the mixture was added ethyl acetate and dil. sodium hydroxide aqueous solution. And the mixture was filtrated on celite. The organic layer was washed with dil. sodium hydroxide aqueous solution, dried, concentrated, and chromatographed on silica gel to give (6Z,8E)-methyl 5,10-dihydroxy-deca-6,8-dienoate.

2) To a tetrahydrofuran suspension (50 ml) of magnesium (6.3 g), was added a tetrahydrofuran solution (10 ml) of trimethyl ortho-4-bromobutyrate (20 g) dropwise at about 25°C. Then, to a tetrahydrofuran solution (400 ml) of (2E,4Z)-ethyl 5-formyl-penta-2,4-dienoate (9.1 g) was added the above reaction mixture dropwise at about -50°C. The mixture was warmed gradually to room temperature, poured into water, and extracted with ether. The extract was dried, and concentrated to give an oil.

To a tetrahydrofuran solution (400 ml) of the above oil, was added lithium tri-sec-butylborohydride (1M solution in tetrahydrofuran; 150 ml) at about -50°C. The mixture was warmed gradually to room temperature, and then acidified to pH 4 - 5 by addition of dil. hydrochloric acid, and extracted with ethyl acetate.

The extract was dried, concentrated and chromatographed on silica gel to give (6Z,8E)-methyl 5,10-dihydroxy-deca-6,8-dienoate.

3) To a tetrahydrofuran solution (240 ml) of naphthalene (7.2 g), was added magnesium chloride (25.9 g) and lithium (3.69 g). The reaction mixture was stirred vigorously at room temperature for 6 hrs. Then to the mixture was added a tetrahydrofuran solution (10 ml) of trimethyl ortho-4-bromobutyrate (20 g) dropwise at about 25°C. Then, to a tetrahydrofuran solution (400 ml) of (2E,4Z)-ethyl 5-formyl-penta-2,4-dienoate (9.1 g) was added the above reaction mixture dropwise at about -50°C. The mixture was warmed gradually to room temperature, and poured into water, extracted with ether. The extract was dried, and concentrated to give an oil.

To a tetrahydrofuran solution (400 ml) of the above oil, was added diisobutylaluminum hydride (1.5M solution in toluene; 100 ml) at about -50°C. The mixture was warmed gradually to room temperature. Then, to the mixture was added ethyl acetate and dil. sodium hydroxide aqueous solution. And the mixture was filtrated on celite. The organic layer was washed with dil. sodium hydroxide aqueous solution, dried, concentrated, and chromatographed on silica gel to give (6Z,8E)-methyl 5,10-dihydroxy-deca-6,8-dienoate.

NMR δ ($CDCl_3$)

3.67 (3H, S), 4.16 (2H, brs), 5.3 - 6.8 (4H, m)

Referential Example 16

To a chloroform solution (20 ml) of (6Z,8E)-methyl 5,10-dihydroxy-deca-6,8-dienoate (0.6 g), was added active manganese dioxide (1.2 g). The reaction mixture was stirred at room temperature. After the starting material had disappeared, the mixture was filtrated on celite, concentrated and chromatographed on silica gel to give (6Z,8E)-methyl 9-formyl-5-hydroxy-nona-6,8-dienoate.

NMR δ ($CDCl_3$)

3.70 (3H, S), 5.7 - 6.4 (3H, m), 7.57 (1H, dd, 15, 12Hz), 9.63 (1H, d, 8Hz)

Referential Example 17

According to the same procedure as that of Referential Example 5, from (6Z,8E)-methyl 9-formyl-5-hydroxy-nona-6,8-dienoate, were obtained the following compounds.

o (6Z,8E,10E)-methyl 5-hydroxy-12-oxo-heptadeca-6,8,10-trienoate

NMR δ ($CDCl_3$)

0.90 (3H, t, 7Hz), 3.69 (3H, S), 4.65 (1H, m), 5.6 - 7.3 (6H, m)

$IR_{cm^{-1}}^{film}$ 3450, 1730, 1660

0183177

o (6Z,8E,10E)-methyl 14-(p-methoxyphenyl)-5-hydroxy-12-oxo-tetradeca-6,8,10-trienoate

NMR δ ($CDCl_3$)

3.67 (3H, S), 3.76 (3H, S), 5.5 - 7.4 (6H, m), 6.76 (2H, d, 8Hz), 7.08 (2H, d, 8Hz)

Referential Example 18

To a tetrahydrofuran solution (240 ml) of naphthalene (7.2 g), was added magnesium chloride (25.9 g) and lithium (3.69 g). The reaction mixture was stirred vigorously at room temperature for 6 hrs. Then to the mixture was added a tetrahydrofuran solution (10 ml) of trimethyl ortho-4-bromobutyrate (20 g) dropwise at about 15°C. Then, to a tetrahydrofuran solution (400 ml) of (2E,4Z)-ethyl 5-formyl-penta-2,4-dienoate (9.1 g) was added the above reaction mixture dropwise at about -50°C. The mixture was warmed gradually to room temperature, and poured into water, extracted with ether. The extact was dried and concentrated to give an oil.

To a tetrahydrofuran solution (400 ml) of the above oil, was added diisobutylaluminum hydride (1.5M solution in toluene; 100 ml) at about -50°C. The mixture was warmed gradually to room temperature. Then, to the mixture was added ethyl acetate and 2N hydrochloric acid. The resulting mixture was stirred, and extracted with ethyl acetate. The extract was dried, concentrated and chromatographed on silica gel to give an oil.

Then, according to the same procedure as Referential Example 16, from the above oil was obtained (6E,8E)-methyl 9-formyl-5-hydroxy-nona-6,8-dienoate.

NMR δ ($CDCl_3$)

3.68 (3H, S), 5.7 - 6.5 (3H, m), 7.11 (1H, dd, 15, 10Hz), 9.57 (1H, d, 8Hz)

Referential Example 19

According to the same procedure as that of Referential Example 5, from (6E,8E)-methyl 9-formyl-5-hydroxy-nona-6,8-dienoate, were obtained the following compounds.

o (6E,8E,10E)-methyl 13-(p-fluorophenoxy)-5-hydroxy-12-oxo-trideca-6,8,10-trienoate

NMR δ ($CDCl_3$)

3.67 (3H, S), 4.66 (2H, S), 5.5 - 7.6 (10H, m)

o (6E,8E,10E)-methyl 14-(3',4'-dichlorophenyl)-5-hydroxy-12-oxo-tetradeca-6,8,10-trienoate

NMR δ ($CDCl_3$)

2.91 (4H, brs), 3.67 (3H, S), 5.5 - 7.5 (9H, m)

o (6E,8E,10E)-methyl 15-(p-methylphenyl)-5-hydroxy-12-oxo-pentadeca-6,8,10-trienoate

NMR δ ($CDCl_3$)

2.31 (3H, S), 3.68 (3H, S), 5.5 - 7.5 (6H, m), 7.08 (4H, S)

o (6E,8E,10E)-methyl 14-(m-trifluoromethylphenyl)-5-hydroxy-12-oxo-tetradeca-6,8,10-trienoate

NMR δ ($CDCl_3$)

2.99 (4H, brs), 3.67 (3H, S), 5.6 - 7.6 (10H, m)

o (6E,8E,10E,14Z)-methyl 5-hydroxy-12-oxo-tricosa-6,8,10,14-tetraenoate

NMR δ ($CDCl_3$)

0.88 (3H, br), 2.4 - 2.8 (4H, m), 3.67 (3H, S), 5.3 - 7.4 (8H, m)

Referential Example 20

To a tetrahydrofuran solution (240 ml) of naphthalene (7.2 g), was added magnesium chloride (25.9 g) and lithium (3.69 g). The reaction mixture was stirred vigorously at room temperature for 6 hrs. Then to the mixture was added a tetrahydrofuran solution (10 ml) of trimethyl ortho-4-bromobutyrate (20 g) dropwise at about 25°C. Then, to a tetrahydrofuran solution (400 ml) of (4Z)-ethyl 5-formyl-4-pentenoate (9.1 g) was added the above reaction mixture dropwise at about -50°C. The mixture was warmed gradually to room temperature, and poured into water, extracted with ether. The extract was dried and concentrated to give an oil.

To a tetrahydrofuran solution (400 ml) of the above oil, was added diisobutylaluminum hydride (1.5M

0183177

solution in toluene; 100 ml) at about -50°C. After the starting material had disappeared, to the mixture was added ethyl acetate and dil. sodium hydroxide aqueous solution. And the mixture was filtrated on celite. The organic layer was washed with dil. sodium hydroxide aqueous solution, dried, concentrated, and chromatographed on silica gel to give an oil.

Then, according to the same procedure as Referential Example 5, from the above oil was obtained (6Z,10E)-methyl 13-cyclohexyl-5-hydroxy-12-oxo-trideca-6,10-dienoate.

NMR δ ($CDCl_3$)

3.68 (3H, S), 4.92 (1H, q, 7Hz), 5.81 (2H, br), 6.12 (1H, d, 15Hz), 6.77 (1H, dt, 15, 7Hz)

Referential Example 21

According to the same procedure as that of Referential Example 20, there were obtained the following compounds.

o (6Z,10E)-methyl 14-ethoxy-5-hydroxy-12-oxo-tetradeca-6,10-dienoate

NMR δ ($CDCl_3$)

1.16 (3H, t, 7Hz), 3.68 (3H, S), 4.89 (1H, brm), 5.79 (2H, brs), 6.17 (1H, d, 15Hz), 6.83 (1H, dt, 15, 7Hz)

o (6Z,10E)-methyl 12-cyclopentyl-5-hydroxy-12-oxo-dodeca-6,10-dienoate

NMR δ ($CDCl_3$)

3.67 (3H, S), 4.91 (1H, brm), 5.80 (2H, br), 6.21 (1H, d, 16Hz), 6.84 (1H, dt, 16, 7Hz)

o (6Z,10E)-methyl 13-methyl-5-hydroxy-12-oxo-heptadeca-6,10-diene-15-ynoate

NMR δ ($CDCl_3$)

1.17 (3H, d, 7Hz), 1.74 (3H, S), 2.92 (1H, six, 7Hz), 3.67 (3H, S), 4.92 (1H, q, 7Hz), 5.82 (2H, br), 6.22 (1H, d, 16Hz), 6.87 (1H, dt, 16, 7Hz)

Example 1

Sodium borohydride (100 mg) was added to a methanol solution (30 ml) of (5E,7E,9E)-methyl 4,11-dioxo-nonadeca-5,7,9-trienoate (800 mg) at 0°C, and the mixture was stirred at 0°C for 2 hrs. Then, to this mixture was added dimethylamine (60 wt.% solution in water: 10 ml). The resulting mixture was stirred at room temperature for 12 hr., poured into water and extracted with ethyl acetate. The extract was dried, concentrated and chromatographed on silica gel to give (5E,7E,9E)-N,N-dimethyl-4,11-dihydroxy-nonadeca-5,7,9-trienamide (400 mg).

NMR δ ($CDCl_3$)

0.88 (3H, t, 6Hz), 2.43 (2H, t, 6Hz), 2.94 (3H, S), 3.00 (3H, S), 4.1 (2H, m), 5.5 - 6.3 (6H, m)

$IR_{cm^{-1}}^{film}$ 3400, 1630

Example 2

According to the same procedure as that of Example 1, there were obtained the following compounds.

o (5E,7E,9E)-N,N-dimethyl-13,17-dimethyl-4,11-dihydroxy-octadeca-5,7,9,16-tetraenamide

NMR δ ($CDCl_3$)

0.93 (3H, d, 6Hz), 1.59 (3H, S), 1.67 (3H, S), 2.48 (2H, brt, 6Hz), 2.94 (3H, S), 3.01 (3H, S), 4.2 (2H, brm), 5.08 (1H, t, 7Hz), 5.5 - 6.4 (6H, m)

$IR^{film}_{cm^{-1}}$ 3400, 1630

o (5E,7E,9E)-13-(p-methoxyphenyl)-4,11-dihydroxy-trideca-5,7,9-trienamide

NMR δ ($CDCl_3$)

2.33 (2H, t, 7Hz), 2.65 (2H, t, 8Hz), 3.80 (3H, S), 4.15 (2H, m), 5.5 - 6.4 (6H, m), 6.82 (2H, d, 8Hz), 7.13 (2H, d, 8Hz)

$IR^{film}_{cm^{-1}}$ 3350, 1660

o (5E,7E,9E)-N,N-tetramethylene-13-(p-methoxyphenyl)-4,11-dihydroxy-trideca-5,7,9-trienamide

NMR δ ($CDCl_3$)

1.9 (4H, m), 2.43 (2H, t, 7Hz), 2.66 (2H, t, 7Hz), 3.4 (4H, m), 3.81 (3H, S), 4.15 (2H, br), 5.5 - 6.4 (6H, m), 6.83 (2H, d, 8Hz), 7.15 (2H, d, 8Hz)

$IR^{film}_{cm^{-1}}$ 3400, 1620

o (5E,7E,9E)-N,N-tetramethylene-13-(3',4'-dichlorophenyl)-4,11-dihydroxy-trideca-5,7,9-trienamide

NMR δ ($CDCl_3$)

1.9 (4H, m), 2.45 (2H, t, 7Hz), 2.68 (2H, t, 7Hz), 3.4 (4H, m), 4.2 (2H, m), 5.5 - 6.4 (6H, brm), 7.0 - 7.5 (3H, m)

$IR^{film}_{cm^{-1}}$ 3400, 1620

o (5E,7E,9E)-N-methyl-13-(m-trifluoromethylphenyl)-4,11-dihydroxy-trideca-5,7,9-trienamide

NMR δ ($CDCl_3$)

2.35 (2H, t, 6Hz), 2.8 (2H, m), 2.78 ($\frac{3}{2}$H, S), 2.83 ($\frac{3}{2}$H, S), 4.2 (2H, br), 5.7 (3H, br), 6.18 (4H, br), 7.40 (4H, brs)

$IR^{film}_{cm^{-1}}$ 3350, 1640

o (5E,7E,9E)-N-methyl-13-(3',4'-dichlorophenyl)-4,11-dihydroxy-trideca-5,7,9-trienamide

NMR δ ($CDCl_3$)

2.33 (2H, t, 6Hz), 2.7 (2H, m), 2.77 ($\frac{3}{2}$H, S), 2.83 ($\frac{3}{2}$H, S), 4.2 (2H, m), 5.7 (3H, br), 6.17 (4H, br, S), 7.24 (3H, S)

$IR^{film}_{cm^{-1}}$ 3300, 1640

o (5E,7E,9E)-N-methyl-12-(p-fluorophenoxy)-4,11-dihydroxy-dodeca-5,7,9-trienamide

NMR δ ($CDCl_3$)

2.30 (2H, t, 6Hz), 2.76 ($\frac{3}{2}$H, S), 2.80 ($\frac{3}{2}$H, S),

3.36 (2H, brd, 6Hz), 4.2 (1H, br), 4.6 (1H, br), 5.8 (3H, br), 6.24 (4H, brs), 6.90 (4H, m)

$IR^{film}_{cm^{-1}}$ 3300, 1640

o (5E,7E,9E)-N,N-dimethyl-14-(p-methylphenyl)-4,11-dihydroxy-tetradeca-5,7,9-trienamide

NMR δ ($CDCl_3$)

2.32 (3H, S), 2.46 (2H, t, 6Hz), 2.60 (2H, t, 6Hz), 2.94 (3H, S), 3.00 (3H, S), 4.2 (2H, m), 5.7 (2H, m), 6.18 (4H, br, S), 7.09 (4H, S)

$IR^{film}_{cm^{-1}}$ 3400, 1630

o (5E,7E,9E)-N,N-dimethyl-13-(p-methoxyphenyl)-4,11-dihydroxy-trideca-5,7,9-trienamide

NMR δ ($CDCl_3$)

2.48 (2H, t, 6Hz), 2.66 (2H, t, 6Hz), 2.96 (3H, S), 3.01 (3H, S), 3.79 (3H, S), 4.2 (2H, br), 5.75 (2H, br), 6.20 (4H, brs), 6.80 (2H, d, 8Hz), 7.11 (2H, d, 8Hz)

$IR^{film}_{cm^{-1}}$ 3400, 1630

o (5E,7E,9E)-N,N-dimethyl-12-cyclohexyl-4,11-dihydroxy-dodeca-5,7,9-trienamide

NMR δ ($CDCl_3$)

2.50 (2H, t, 6Hz), 2.98 (3H, S), 3.03 (3H, S), 4.28 (2H, q, 6Hz), 5.7 (2H, m), 6.22 (4H, brs)

$IR^{film}_{cm^{-1}}$ 3350, 1630

o (5E,7E,9E)-N,N-dimethyl-13-(p-dimethylaminophenyl)-4,11-dihydroxy-trideca-5,7,9-trienamide

NMR δ ($CDCl_3$)

2.90 (6H, S), 2.95 (3H, S), 3.00 (3H, S), 4.2 (2H, m), 5.7 (2H, m), 6.18 (4H, brs), 6.68 (2H, d, 9Hz), 7.06 (2H, d, 9Hz)

$IR^{film}_{cm^{-1}}$ 3400, 1620

o (5E,7E,9E)-N,N-dimethyl-11-cyclopentyl-4,11-dihydroxy-undeca-5,7,9-trienamide

NMR δ ($CDCl_3$)

2.50 (2H, t, 6Hz), 2.97 (3H, S), 3.02 (3H, S), 3.96 (1H, t, 7Hz), 4.28 (1H, q, 7Hz), 5.75 (2H, m), 6.20 (4H, brs)

$IR^{film}_{cm^{-1}}$ 3350, 1620

o (5E,7E,9E)-N,N-dimethyl-13-methyl-4,11-dihydroxy-heptadeca-5,7,9-trienamide

NMR δ ($CDCl_3$)

2.50 (2H, t, 6Hz), 2.98 (3H, S), 3.03 (3H, S), 4.2 (2H, m), 5.7 (2H, m), 6.20 (4H, brs)

$IR^{film}_{cm^{-1}}$ 3400, 1630

o (5E,7E,9E)-N,N-dimethyl-13-(o-methoxyphenyl)-4,11-dihydroxy-trideca-5,7,9-trienamide

NMR δ ($CDCl_3$)

2.46 (2H, t, 6Hz), 2.71 (2H, t, 6Hz), 2.93 (3H, S), 2.99 (3H, S), 3.81 (3H, S), 4.2 (2H, br),

5.75 (2H, br), 6.16 (4H, brs), 6.7 - 7.3 (4H, m)

$IR^{film}_{cm^{-1}}$ 3400, 1620

o (5E,7E,9E)-N,N-dimethyl-13-(3',4',5'-trimethoxyphenyl)-4,11-dihydroxy-trideca-5,7,9-trienamide

NMR δ ($CDCl_3$)

2.47 (2H, t, 6Hz), 2.66 (2H, t, 6Hz), 2.94 (3H, S), 3.01 (3H, S), 3.82 (3H, S), 3.85 (6H, S), 4.2 (2H, br), 5.75 (2H, br), 6.18 (4H, brs), 6.42 (2H, S)

$IR^{film}_{cm^{-1}}$ 3400, 1620

o (5E,7E,9E)-N,N-tetramethylene-13-(m-methoxyphenyl)-4,11-dihydroxy-trideca-5,7,9-trienamide

NMR δ ($CDCl_3$)

2.41 (2H, t, 6Hz), 2.70 (2H, brt, 6Hz), 3.42 (4H, br), 3.78 (3H, S), 4.2 (2H, br), 5.8 (2H, br), 6.18 (4H, brs), 6.7 - 7.4 (4H, m)

$IR^{film}_{cm^{-1}}$ 3400, 1620

o (5E,7E,9E)-N,N-tetramethylene-13-(3',4'-dimethoxyphenyl)-4,11-dihydroxy-trideca-5,7,9-trienamide

NMR δ ($CDCl_3$)

3.43 (4H, br), 3.85 (6H, S), 4.2 (2H, br), 5.7 (2H, br), 6.20 (4H, br), 6.73 (3H, br)

$IR^{film}_{cm^{-1}}$ 3400, 1620

0183177

Example 3

According to the same procedure as that of Example 1, from (5E,9E)-methyl 4,11-dioxo-nonadeca-5,9-dienoate was obtained the following compound.

o (5E,9E)-N,N-dimethyl-4,11-dihydroxy-nonadeca-5,9-dienamide

NMR δ ($CDCl_3$)

0.88 (3H, brt), 2.15 (4H, m), 2.48 (2H, t, 7Hz), 2.96 (3H, S), 3.03 (3H, S), 4.09 (2H, m), 5.57 (4H, m)

$IR^{film}_{cm^{-1}}$ 3400, 1630

Example 4

According to the same procedure as that of Example 1, from (6E,8E,10E)-methyl 13-(p-fluorophenoxy)-5,12-dioxo-trideca-6,8,10-trienoate, was obtained (6E,8E,10E)-N,N-tetramethylene-13-(p-fluorophenoxy)-5,12-dihydroxy-trideca-6,8,10-trienamide.

NMR δ ($CDCl_3$)

2.30 (2H, t, 6Hz), 3.42 (6H, m), 4.16 (1H, m), 4.56 (1H, m), 5.8 (2H, m), 6.21 (4H, brs), 6.87 (4H, m)

$IR^{film}_{cm^{-1}}$ 3400, 1620

Example 5

According to the same procedure as that of Example 4, there were obtained the following compounds.

o (6E,8E,10E)-N,N-dimethyl-14-(m-trifluoromethylphenyl)-5,12-dihydroxy-tetradeca-6,8,10-trienamide 

NMR δ ($CDCl_3$)

2.34 (2H, t, 6Hz), 2.79 (2H, t, 7Hz), 2.94 (3H, S), 3.00 (3H, S), 4.18 (2H, br), 5.75 (2H, br), 6.16 (4H, brs), 7.39 (4H, brs)

$IR^{film}_{cm^{-1}}$ 3350, 1620

o (6E,8E,10E,14Z)-N-methyl-5,12-dihydroxy-tricosa-6,8,10,14-tetraenamide

NMR δ ($CDCl_3$)

0.88 (3H, brt), 2.22 (2H, t, 7Hz), 2.79 (3H, br), 4.2 (2H, br), 5.35 (2H, br), 5.8 (2H, br), 6.19 (4H, brs)

$IR^{film}_{cm^{-1}}$ 3300, 1640

o (6E,8E,10E)-N,N-tetramethylene-14-(3',4'-dichlorophenyl)-5,12-dihydroxy-tetradeca-6,8,10-trienamide

NMR δ ($CDCl_3$)

2.30 (2H, t, 6Hz), 2.69 (2H, t, 6Hz), 3.43 (4H, m), 4.2 (2H, br), 5.8 (2H, br), 6.16 (4H, brs), 6.9 - 7.4 (3H, m)

$IR^{film}_{cm^{-1}}$ 3400, 1620

o (6E,8E,10E)-N,N-dimethyl-15-(p-methylphenyl)-5,12-dihydroxy-pentadeca-6,8,10-trienamide

NMR δ ($CDCl_3$)

2.28 (3H, S), 2.92 (3H, S), 2.98 (3H, S), 4.15 (2H, m), 5.7 (2H, br), 6.12 (4H, brs),

7.06 (4H, S)

$IR^{film}_{cm^{-1}}$ 3400, 1630

Example 6

To an ethanol solution (5 ml) of sodium borohydride (50 mg) was added an ethanol solution (0.5 ml) of (6Z,8E,10E)-methyl 5-hydroxy-12-oxo-heptadeca-6,8,10-trienoate (150 mg) at -70°C. The mixture was warmed gradually to room temperature. Then to the mixture was added ammonium chloride aqueous solution. The mixture was extracted with ethyl acetate. The extract was dried, concentrated, and chromatographed on silica gel to give (6Z,8E,10E)-methyl 5,12-dihydroxy-heptadeca-6,8,10-trienoate.

NMR δ ($CDCl_3$)

0.89 (3H, br), 3.67 (3H, S), 5.4 - 6.5 (6H, m),

$IR^{film}_{cm^{-1}}$ 3400, 1730

UV ($CH_3OH$) 258, 269, 280 nm

Example 7

(6Z,8E,10E)-methyl 5,12-dihydroxy-heptadeca-6,8,10-trienoate (45 mg) was dissolved in methanol containing ammonia. The mixture was stirred at room temperature for 10 days, and concentrated, and chromatographed on silica gel to give (6Z,8E,10E)-5,12-dihydroxy-heptadeca-6,8,10-trienamide.

NMR δ ($CDCl_3$)

0.87 (3H, brt, 6Hz), 5.6 - 6.5 (6H, m)

$IR^{film}_{cm^{-1}}$ 3350, 1660

UV ($CH_3OH$) 260, 269, 280 nm

Example 8

According to the same procedures as that of Example 6 and Example 7, there were obtained the following compounds.

o (6Z,8E,10E)-N,N-dimethyl-14-(p-methoxyphenyl)-5,12-dihydroxy-tetradeca-6,8,10-trienamide

NMR δ ($CDCl_3$)

2.87 (3H, S), 2.92 (3H, S), 3.72 (3H, S),
5.3 - 6.4 (6H, m), 6.72 (2H, d, 8Hz),
7.03 (2H, d, 8Hz)

$IR^{film}_{cm^{-1}}$ 3400, 1630

o (6Z,10E)-13-cyclohexyl-5,12-dihydroxy-trideca-6,10-dienamide

NMR δ ($CDCl_3$)

2.28 (4H, br), 4.83 (2H, br), 5.58 (2H, br),
5.78 (2H, br)

$IR^{film}_{cm^{-1}}$ 3350, 1660

o (6Z,10E)-14-ethoxy-5,12-dihydroxy-tetradeca-6,10-dienamide

NMR δ ($CDCl_3$)

1.18 (3H, t, 7Hz), 4.85 (2H, br), 5.80 (4H, br)

$IR^{film}_{cm^{-1}}$ 3350, 1660

Example 9

According to the same procedure as that of Example 8, (6E,8E,10E)-methyl 13-(p-fluorophenoxy)-5-hydroxy-12-oxo-trideca-6,8,10-trienoate, (6E,8E,10E)-methyl 14-(3',4'-dichlorophenyl)-5-hydroxy-12-oxo-tetradeca-6,8,10-trienoate, (6E,8E,10E)-methyl 15-(p-methylphenyl)-5-hydroxy-12-oxo-pentadeca-6,8,10-trienoate, (6E,8E,10E)-methyl 14-(m-trifluoromethylphenyl)-5-hydroxy-12-oxo-tetradeca-6,8,10-trienoate, and (6E,8E,10E,14Z)-methyl 5-hydroxy-12-oxo-tricosa-6,8,10,14-tetraenoate, were converted respectively into (6E,8E,10E)-N,N-tetramethylene-13-(p-fluorophenoxy)-5,12-dihydroxy-trideca-6,8,10-trienamide, (6E,8E,10E)-N,N-tetramethylene-14-(3',4'-dichlorophenyl)-5,12-dihydroxy-tetradeca-6,8,10-trienamide, (6E,8E,10E)-N,N-dimethyl 15-(p-methylphenyl)-5,12-dihydroxy-pentadeca-6,8,10-trienamide, (6E,8E,10E)-N,N-dimethyl-14-(m-trifluoromethylphenyl)-5,12-dihydroxy-tetradeca-6,8,10-trienamide, and (6E,8E,10E,14Z)-N-methyl-5,12-dihydroxy-tricosa-6,8,10,14-tetraenamide.

It was confirmed by their spectrum data, $^{1}$H-NMR and IR, that the obtained compounds were the same as the compounds obtained in Example 4 and Example 5.

Example 10

According to the same procedure as that of Example 6, there were obtained the following compounds.

o (6Z,10E)-methyl 12-cyclopentyl-5,12-dihydroxy-dodeca-6,10-dienoate

NMR δ ($CDCl_3$)

3.67 (3H, S), 4.85 (2H, br), 5.56 (2H, m), 5.78 (2H, brs)

$IR^{film}_{cm^{-1}}$ 3400, 1740

o (6Z,10E)-methyl 13-methyl-5,12-dihydroxy-hetadeca-6,10-diene-15-ynoate

NMR δ ($CDCl_3$)

0.94 ($\frac{3}{2}$H, d, 6Hz), 0.97 ($\frac{3}{2}$H, d, 6Hz), 1.77 ($\frac{3}{2}$H, S), 1.79 ($\frac{3}{2}$H, S), 3.66 (3H, S), 4.82 (2H, br), 5.68 (2H, br), 5.79 (2H, brs)

$IR^{film}_{cm^{-1}}$ 3400, 1740

The products obtained in the above-mentioned Examples are summarized as follows in the table:

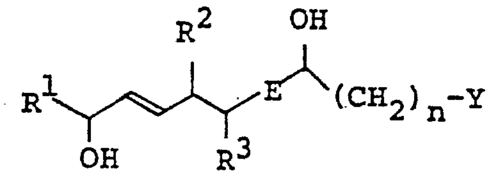

| Compound No. | $R^1$ | $R^2$, $R^3$ | E | n | Y |
|---|---|---|---|---|---|
| 1 | 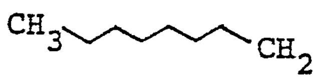 | single linkage | *trans* | 2 | $CONMe_2$ |
| 2 | 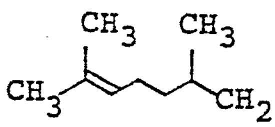 | " | " | " | " |
| 3 | 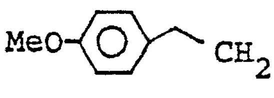 | " | " | " | $CONH_2$ |
| 4 | " | " | " | " | 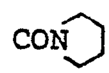 |
| 5 | 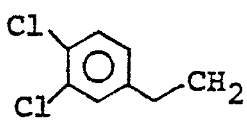 | " | " | " | " |
| 6 | 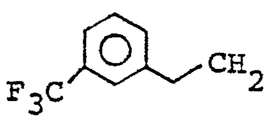 | " | " | " | CONHMe |
| 7 | 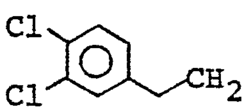 | " | " | " | " |
| 8 | 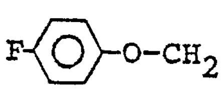 | " | " | " | " |
| 9 | 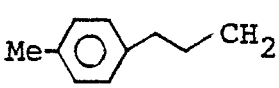 | " | " | " | $CONMe_2$ |
| 10 | 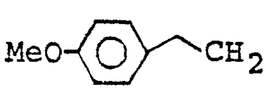 | " | " | " | " |

(cont'd)

| | | | | | |
|---|---|---|---|---|---|
| 11 | 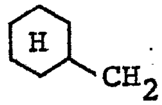 | single linkage | *trans* | 2 | $CONMe_2$ |
| 12 | 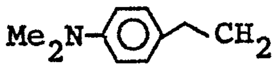 | " | " | " | " |
| 13 | 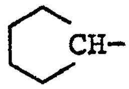 | " | " | | " |
| 14 | 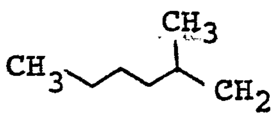 | " | " | " | " |
| 15 | 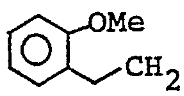 | " | " | " | " |
| 16 | 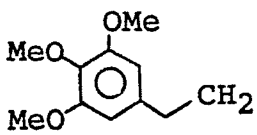 | " | " | " | " |
| 17 | 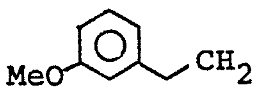 | " | " | " | CON 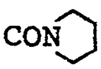 |
| 18 | 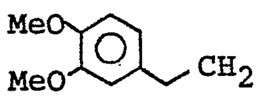 | " | " | " | " |
| 19 | 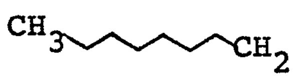 | hydrogen atoms | " | " | $CONMe_2$ |
| 20 | 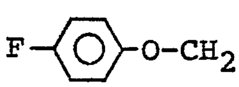 | single linkage | " | 3 | CON 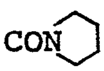 |
| 21 | 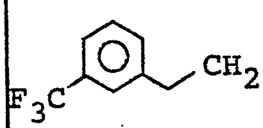 | " | " | " | $CONMe_2$ |
| 22 | 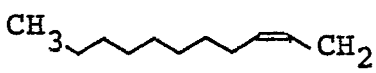 | " | " | " | CONHMe |
| 23 | 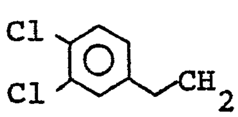 | " | " | " | CON 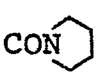 |

(cont'd) 

| | | | | | |
|---|---|---|---|---|---|
| 24 | 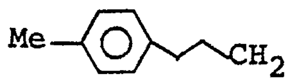 | single linkage | *trans* | 3 | $CONMe_2$ |
| 25 | 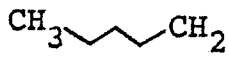 | " | *cis* | " | $CO_2Me$ |
| 26 | " | " | " | " | $CONH_2$ |
| 27 | 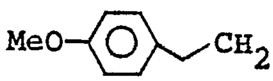 | " | " | " | $CONMe_2$ |
| 28 | 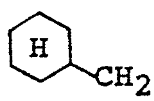 | hydrogen atoms | " | " | $CONH_2$ |
| 29 | 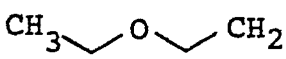 | " | " | " | " |
| 30 | 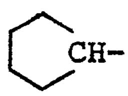 | " | " | " | $CO_2Me$ |
| 31 | 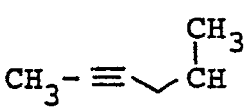 | " | " | " | " |

WHAT IS CLAIMED IS:

1. A compound of the formula:

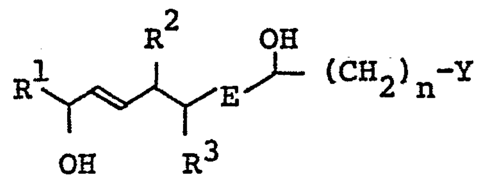

wherein Y is a free or esterified carboxyl group, or a group of the formula:

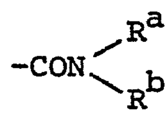

(wherein $R^a$ and $R^b$ are each independently a hydrogen atom, a $C_1$-$C_4$ alkyl group, a $C_3$-$C_7$ cycloalkyl group, a benzyl group, a phenyl group, a phenyl group substituted with a halogen atom or a $C_1$-$C_4$ alkyl group, or, when taken together with the adjacent nitrogen atom, they represent a 5 to 7 membered saturated heterocyclic group); $R^1$ is a $C_1$-$C_{12}$ alkyl group, a $C_2$-$C_{12}$ alkenyl group, a $C_2$-$C_{12}$ alkynyl group, a $C_3$-$C_{10}$ cycloalkyl group, a $C_4$-$C_{10}$ cycloalkenyl group, a hydroxy $C_1$-$C_{12}$ alkyl group, an $C_1$-$C_{12}$ alkyl group substituted with a group of the formula:

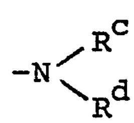

(wherein $R^c$ and $R^d$ are each independently a hydrogen atom, or a $C_1$-$C_4$ alkyl group), a $C_3$-$C_{10}$ heterocyclic group, a phenyl group optionally substituted with one to three substituents selected from the group consisting of

halogen atom, hydroxy group, $C_1$-$C_4$ alkyl group, trifluoromethyl group, $C_1$-$C_4$ alkoxy group, and group of the formula: 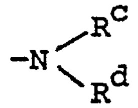 (wherein $R^c$ and $R^d$ are as defined above) or a group of the formula: A-B [wherein A is a $C_1$-$C_7$ alkylene chain and B is a $C_3$-$C_{10}$ cycloalkyl group, a $C_4$-$C_{10}$ cycloalkenyl group, a $C_1$-$C_{12}$ alkoxy group, a $C_1$-$C_{12}$ alkylthio group, a $C_3$-$C_{10}$ cycloalkoxy group, a $C_4$-$C_{10}$ cycloalkenyloxy group, a $C_3$-$C_{10}$ heterocyclic group, or a phenyl or phenoxy group optionally substituted with one to three substituents selected from the group consisting of halogen atom, hydroxy group, $C_1$-$C_4$ alkyl group, group of the formula: 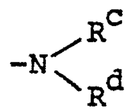 (wherein $R^c$ and $R^d$ are as defined above), trifluoromethyl group, $C_1$-$C_4$ alkylthio group and $C_1$-$C_4$ alkoxy group]; n is 2, 3 or 4; $R^2$ and $R^3$ are hydrogen atoms or, when taken together, they mean a single linkage to form a *trans* double bond between the adjacent carbon atoms, and E is a *cis* or *trans* vinylene group; provided that when n is 3 and $R^1$ is a n-octyl group, a 2-octenyl group or a 2,5-octadienyl group, $R^2$ and $R^3$ are hydrogen atoms, and that when n is 2, E is a *trans* vinylene group.

2. The compound according to Claim 1, wherein Y is a group of the formula:

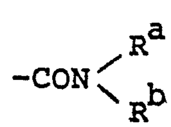

(wherein $R^a$ and $R^b$ are each independently a hydrogen atom, a $C_1$-$C_4$ alkyl group, or, when taken together with the adjacent nitrogen atom, they represent a 5 to 7 membered saturated heterocyclic group); $R^1$ is a $C_1$-$C_{12}$ alkyl group, a $C_2$-$C_{12}$ alkeny group, a $C_2$-$C_{12}$ alkynyl group, a $C_3$-$C_{10}$ cycloalkyl group, or a group of the formula: A-B [A is a $C_1$-$C_7$ alkylene chain and B is a $C_3$-$C_{10}$ cycloalkyl group, a $C_1$-$C_{12}$ alkoxy group, or a phenyl or phenoxy group optionally substituted with one to three substituents selected from the group consisting of halogen atom, $C_1$-$C_4$ alkyl group, group of the formula: 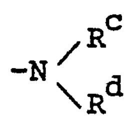 (wherein $R^c$ and $R^d$ are each independently a $C_1$-$C_4$ alkyl group), trifluoromethyl group, and $C_1$-$C_4$ alkoxy group].

3. The compound according to Claim 1, which is represented by the formula:

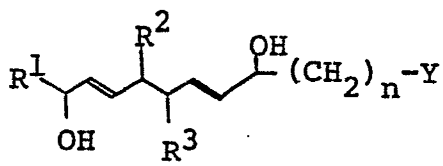

wherein $R^1$, $R^2$, $R^3$, n and Y are each as defined in Claim 1.

4. The compound according to Claim 1 or 2 wherein E is a *trans* vinylene group; and $R^2$ and $R^3$ are combined to mean a single linkage to form a *trans* double bond between the adjacent carbon atoms.

5. The compound according to Claim 1 or 2 wherein E is a *cis* vinylene group; n is 3 or 4; and $R^2$ and $R^3$

mean a single linkage to form a *trans* double bond between the adjacent carbon atoms.

6. The compound according to Claim 1, which is represented by the formula:

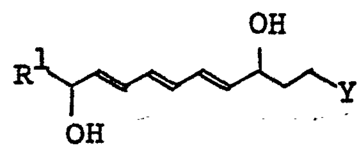

wherein $R^1$ and Y are each as defined in Claim 1.

7. The compound according to Claim 1, which is represented by the formula:

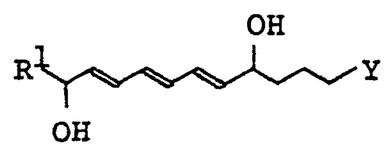

wherein $R^1$ and Y are each as defined in Claim 1.

8. The compound according to Claim 2, which is represented by the formula:

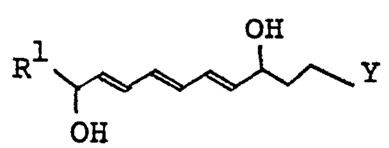

wherein $R^1$ and Y are each as defined in Claim 2.

9. The compound according to Claim 2, which is represented by the formula:

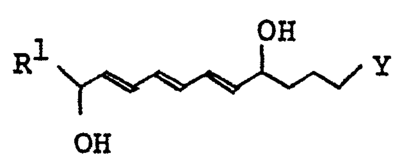

wherein $R^1$ and Y are each as defined in Claim 2.

10. The compound according to Claim 1, wherein n is 3 or 4.

11. A compound according to Claim 1, wherein $R^1$ is a group of the formula: A-B.

12. A compound according to Claim 11, wherein n is 3.

13. The compound according to Claim 1, wherein E is a *trans* vinylene group, and n=2.

14. (5E,7E,9E)-N,N-dimethyl-4,11-dihydroxy-nonadeca-5,7,9-trienamide.

15. (5E,7E,9E)-N,N-dimethyl-4,11-dihydroxy-13-(4'-methoxyphenyl)-trideca-5,7,9-trienamide.

16. (5E,7E,9E)-N,N-tetramethylene-4,11-dihydroxy-13-(4'-methoxyphenyl)-trideca-5,7,9-trienamide.

17. (6Z,8E,10E)-N,N-dimethyl-5,12-dihydroxy-14-(4'-methoxyphenyl)-tetradeca-6,8,10-trienamide.

18. (5E,7E,9E)-N,N-tetramethylene-4,11-dihydroxy-13-(3',4'-dichlorophenyl)-trideca-5,7,9-trienamide.

19. A process for producing a compound of the formula:

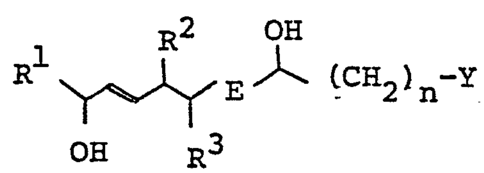

wherein $R^1$, $R^2$, $R^3$, E, n and Y are each as defined in Claim 1, and its non-toxic pharmaceutically acceptable salt, which comprises reacting a compound of the formula:

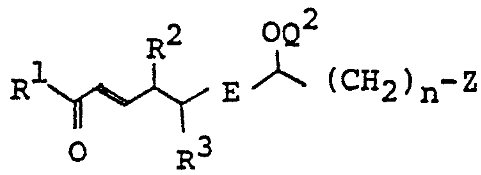

wherein $R^1$, $R^2$, $R^3$, E and n are as defined above; $Q^2$ is a hydrogen atom or an acyl group; and Z is an esterified carboxyl group or a group of the formula:

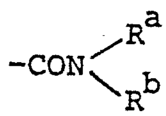

(wherein $R^a$ and $R^b$ are as defined in Claim 1), with a reducing agent optionally followed by deprotection of a protected hydroxyl group, hydrolysis of an ester group, esterification of a carboxyl group, amidation of a free or esterified carboxyl group, and/or transesterification.

20. A process for producing a compound of the formula:

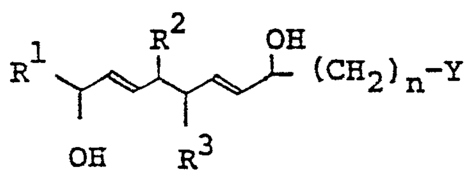

wherein $R^1$, $R^2$, $R^3$, n and Y are each as defined in Claim 1, or its non-toxic pharmaceutically acceptable salt, which comprises reacting a compound of the formula:

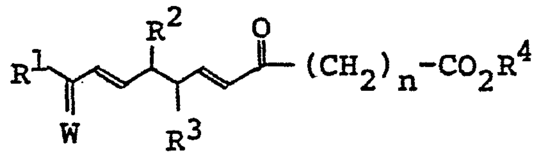

wherein $R^1$, $R^2$, $R^3$ and n are each as defined in Claim 1, and $R^4$ is a $C_1$-$C_4$ alkyl group, W is an oxygen atom, or a group of the formula:

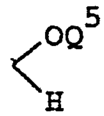

($Q^5$ is an acyl group or a substituted alkoxymethyl group which forms acetal with the adjoining oxygen atom), with a reducing agent optionally followed by deprotection of a protected hydroxyl group, hydrolysis of an ester group, esterification of a carboxyl group, amidation of a free or esterified carboxyl group, and/or transesterification.

21. A process for producing a compound of the formula:

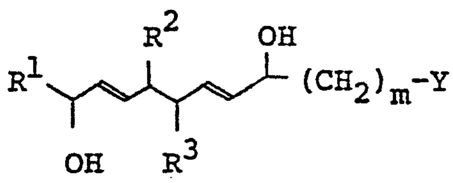

wherein m is 3 or 4, and $R^1$, $R^2$, $R^3$ and Y are each as defined in Claim 1, and its non-toxic pharmaceutically acceptable salt, which comprises reacting a compound of the formula:

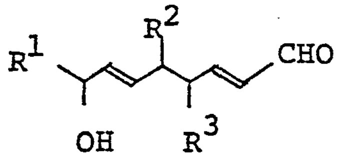

wherein $R^1$, $R^2$ and $R^3$ are each as defined in Claim 1, with an organometallic compound of the formula:

$$M^1-(CH_2)_m-C(OR^5)_3$$

wherein $M^1$ is a lithium atom or a group of the formula: MgX (X is a chlorine atom, a bromine atom or an iodine atom); $R^5$ is a $C_1-C_4$ alkyl group, and m is 3 or 4, followed by transformation of an orthoester group to an ester group, and optionally followed by hydrolysis of an ester group, esterification of a carboxyl group, amidation of a free or esterified carboxyl group and/or transesterification.

22. A compound of the formula:

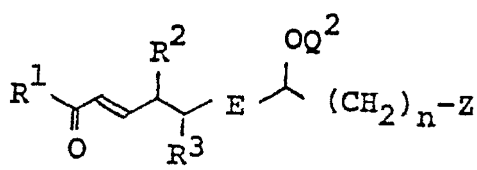

wherein $R^1$, $R^2$, $R^3$, E, $Q^2$, n and Z are each as defined in Claim 19.

23. A compound of the formula:

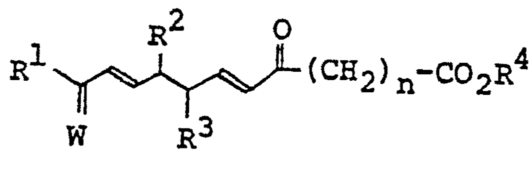

wherein $R^1$, $R^2$, $R^3$, $R^4$, W and n are each as defined in Claim 20.

24. A pharmaceutical composition which comprises a compound as claimed in any of Claims 1 to 18 and a pharmaceutically acceptable carrier or diluent.

25. Use of a compound as claimed in any of Claims 1 to 18 for the preparation of an anti-leucotriene $B_4$ drug.

European Patent Office

# EUROPEAN SEARCH REPORT

0183177

Application number

EP 85 11 4733

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| | | | C 07 C 103/56<br>C 07 C 103/30<br>A 61 K 31/16<br>C 07 D 295/18<br>A 61 K 31/23<br>A 61 K 31/19<br>C 07 C 69/732<br>C 07 C 69/734<br>C 07 C 59/42<br>C 07 C 59/46 |
| X | PROSTAGLANDINS, vol. 23, no. 5, May 1982, pages 631-641; R.D.R. CAMP et al.: "Neutrophil aggregating and chemokinetic properties of a 5,12,20-trihydroxy-6,8,10,14-eicosatetraenoic acid isolated from human leukocytes"<br>* Pages 631-632 *<br>--- | 1,3,4,6,13 | |
| X | CHEMICAL ABSTRACTS, vol. 97, 1982, no. 33932m, Columbus, Ohio, US; J.A. LINDGREN et al.: "Formation of novel biologically active leukotrienes by omega-oxidation in human leukocyte preparations", & ADV. PROSTAGLANDIN, THROMBOXANE, LEUKOTRIENE RES. 1982, 9(Leukotrienes Other Lipoxygenase Prod.), 53-60<br>* Abstract *<br>--- | 1,3,4,6,13 | TECHNICAL FIELDS SEARCHED (Int. Cl.4) |
| | | | C 07 C 103/00<br>A 61 K 31/00<br>C 07 D 295/00 |
| X | CHEMICAL ABSTRACTS, vol. 97, 1982, page 469, no. 53710r, Columbus, Ohio, US; A.G. CASTLE et al.: "Absence of 5'-nucleotidase in porcine polymorphonuclear leukocyte membranes", & BIOCHIM. BIOPHYS. ACTA 1982, 688(2), 637-44<br>* Abstract *<br>--- -/- | 1,3,4,6,13 | |

The present search report has been drawn up for all claims

| Place of search | Date of completion of the search | Examiner |
|---|---|---|
| THE HAGUE | 24-02-1986 | PAUWELS G.R.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82

European Patent Office

# EUROPEAN SEARCH REPORT

0183177

Application number

EP 85 11 4733



## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 97, 1982, page 523, no. 142973w, Columbus, Ohio, US; H.J. SHOWELL et al.: "Inhibition of leukotriene B4-induced neutrophil degranulation by leukotriene B4-dimethylamide", & BIOCHEM. BIOPHYS. RES. COMMUN. 1982, 106(3), 741-7<br>----- | 1-25 | |
| | | | TECHNICAL FIELDS SEARCHED (Int. Cl.4) |

The present search report has been drawn up for all claims

| Place of search | Date of completion of the search | Examiner |
|---|---|---|
| THE HAGUE | 24-02-1986 | PAUWELS G.R.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82